# EUROPEAN PATENT APPLICATION

(11) **EP 1 905 766 A1**
(43) Date of publication of application: **02.04.2008**
(21) Application number: 06780844.4
(22) Date of filing: 30.06.2006
(51) Int. Cl.: C07D 309/10

(54) **GLYCOLIPID DERIVATIVE AND THERAPEUTIC AGENT COMPRISING THE SAME AS ACTIVE INGREDIENT**

(30) Priority: 01.07.2005 JP 2005194116; 13.09.2005 JP 2005265716
(71) Applicant: Japan as represented by President of National Center of Neurology and Psychiatry, Kodaira-shi, Tokyo 187-8551 (JP); Asubio Pharma Co., Ltd., Minato-ku Tokyo 107-8541 (JP)
(72) Inventor: MIYAKE, Sachiko, 1640001 (JP); YAMAMURA, Takashi, 1670051 (JP); TOBA, Tetsuya, 5690814 (JP)
(74) Representative: Stoner, Gerard Patrick
(86) International application number: PCT/JP2006/313519
(87) International publication number: WO 2007/004705

(57) **Abstract**

Glycolipid derivatives having the formula (I): 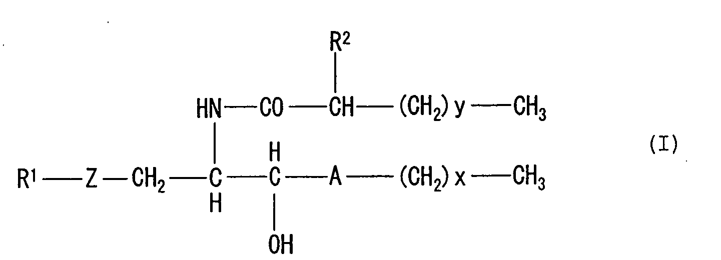 wherein R¹ indicates an aldopyranose residue, R² indicates a hydrogen atom or hydroxyl group, A indicates -CH₂-, - CH(OH)-CH₂- or -CH=CHCH₂-, Z indicates -O- or -CH₂-, when Z is -O- and x is an integer of 4 to 16, y indicates an integer of 26 to 35, when Z is -O- and x indicates an integer of 17 to 25, y indicates an integer of 0 to 35, when Z is -CH₂- and x indicates an integer of 4 to 15, y indicates an integer of 26 to 35, and when Z is -CH₂- and x indicates an integer of 16 to 25, y indicates an integer of 0 to 35 and a drug containing the glycolipid derivative for treatment of autoimmune arthritis and other autoimmune disease, bronchial asthma and other allergic diseases or diseases in which NKT cells or stimulation of NKT cells is known to be participating in the deterioration of conditions.

## Description

### TECHNICAL FIELD

The present invention relates to a novel glycolipid derivative useful for multiple sclerosis, myasthenia gravis, chronic rheumatoid arthritis, systemic lupus erythematosus, Sjogren syndrome, systemic scleroderma, polymyositis, dermatomyositis, insulin-dependent diabetes, idiopathic thrombocytopenic purpura, Hashimoto's thyroiditis, Basedow's disease, pernicious anemia, Addison's disease, atrophic gastritis, hemolytic anemia, Crohn's disease, ulcerative colitis, autoimmune hepatitis, pemphigus, pemphigoid, vasculitis syndrome, autoimmune hemolytic anemia, Goodpasture's syndrome, Lupus nephritis, and other autoimmune diseases and bronchial asthma, atopic asthma, atopic dermatitis, allergic rhinitis, urticarial eruption, hayfever, drug allergy, contact dermatitis, and other allergic diseases and organ transplant rejection and the pharmacologically acceptable hydrate and solvate thereof and a drug containing the same.

### BACKGROUND ART

An immune system inherently distinguishes cells, etc.to of itself and not of itself, excludes cells etc. not of itself by an immunoresponse, and induces immunological unresponsiveness for cells etc. of itself so as to maintain the homeostasis of the body. Further, a condition where the maintenance of the immunological unresponsiveness for cells etc. of itself collapses and the immune system attacks cells etc. of itself may be considered an "autoimmune disease", while a condition where the immunoresponse caused against antigens not of itself (foreign matter) causes problems in the body due to the excessive response may be considered an "allergy".

The conventional method of treatment of autoimmune disease and allergic diseases has mainly been the nonspecific immunosuppressive treatment of glucocorticoids and immunosuppressives. However, these treatment methods have many side effects. Development of autoantigen-specific immunosuppressives has been earnestly desired. In recent years, the method of peptide treatment of autoantigens has been experimented with, but peptides are provided by major histocompatibility gene complexes (MHC) rich in polymorphism, so there are remarkable individual differences. While there have been cases of improvement, there have also been cases of greater deterioration. The result in the end was difficulty in clinical application.

NKT cells are lymphocytes which express both NK cell markers (NKT receptors) and T-cell antigen receptors (TCR). While T-cells recognize peptides bound to MHC, NKT cells recognize glycolipid derivatives presented by non-polymorphic CD1d molecules as antigens and produces a large amount of cytokines in an extremely short period of time, when stimulated from TCR.

For example, the α-galactosyl ceramide (α-GC) of formula (II): 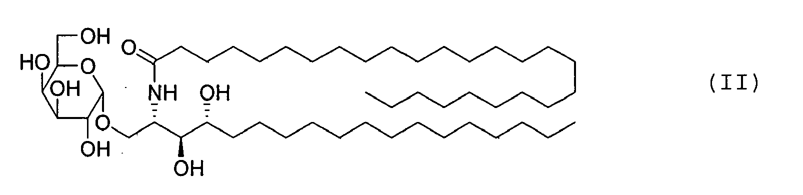 has been reported as the first glycolipid derivative ligand for activating CD1d restricted NKT cells and clarified that it expresses an antitumor activity and immunostimulatory action (see Nonpatent Document 1 and Patent Document 1). Further, the inventors reported that the OCH of the formula (III) shortened in length of the carbon chain of the sphingosine base of α-GC: 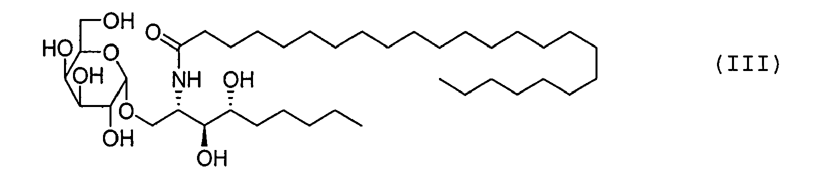 biases the Th1/Th2 immune balance toward Th2 and exhibits high effectiveness in the animal model of multiple sclerosis: murine experimental autoimmune encephalomyelitis (EAE) and the animal model of rheumatoid arthritis: collagen induced arthritis (CIA) (see Nonpatent Documents 2 to 4 and Patent Document 2).

That is, the expression of the action of the OCH of the formula (III) in the above autoimmune disease animal model can be explained based on the "active suppression" by the production of Th2 type inhibitory cytokines from the NKT cells in charge of immune response.

On the other hand, it is reported that NKT cells function as effector cells participating in the deterioration of disease conditions in arthritis and other autoimmune disease models or bronchial asthma models (see Nonpatent Document 5). Therefore, if a drug therapy suppressing the functions of NKT cells in such disease conditions could be established, it would lead to not only the prevention and treatment of autoimmune diseases, but also the treatment of allergic diseases. However, no effective drug treatment using the suppression of the functions of NKT cells as the mechanism for action has yet been known.
Patent Document 1: Japanese Patent No. 3088461
Patent Document 2: WO2003/016326
Nonpatent Document 1: T. Kawano et al., Proc. Natl. Acad. Sci. USA. 1998, 95, 5690
Nonpatent Document 2: K. Miyamoto et al., Nature 2001, 413, 531
Nonpatent Document 3: A. Chiba et al., Arthritis Rheum. 2004, 50, 305
Nonpatent Document 4: T. Yamamura et al., Curr. Top. Med. Chem. 2004, 4, 561
Nonpatent Document 5: O. Akbari et al., Curr. Opin. Immunol. 2003, 15, 627

### DISCLOSURE OF THE INVENTION

In view of the above circumstances, the object of the present invention is to provide a high safety drug for improvement or treatment suppressing the functions of NKT cells and, thereby, effective of autoimmune diseases, allergic diseases, and diseases in which NKT cells are known to contribute to the deterioration, by suppressing the functions of NKT cells.

The another object of the present invention is to provide a novel glycolipid derivative functioning as a ligand of CD1d restricted NKT cells, but induces practically no cytokines such as IL-4 and IFN-γ, or other cytokine production from the NKT cells and useful as the above pharmaceuticals and their pharmacologically acceptable salts, hydrates, and solvates.

The inventors have synthesized glycolipid derivatives capable of controlling the autoimmune response and have been working on the development of a drug for treating autoimmune diseases (W02003/016326; WO2004/072091; Karl O. A. Yu et al., Proc. Natl. Acad. Sci. USA, 2005, 102, 3383). Among these, the inventors found that, in a derivative having a longer length of the carbon chain of the sphingosine base of the glycolipid than α-GC(II) or a derivative with a longer length of the long chain fatty acid, surprisingly a strong suppressive effect of the antibody-induced arthritis is expressed by a glycolipid derivative having the formula (I): 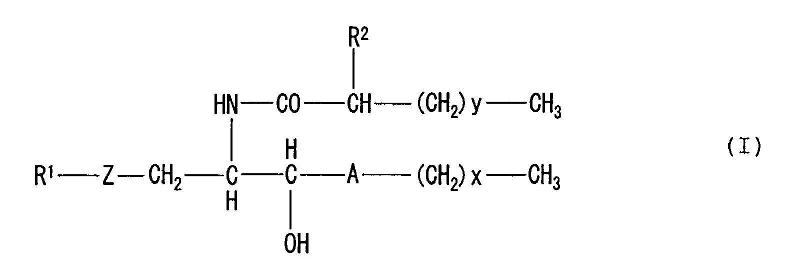 wherein R¹ indicates an aldopyranose residue, R² indicate a hydrogen atom or hydroxyl group, A indicates -CH₂-, -CH(OH)-CH₂- or -CH=CHCH₂-, Z indicates -O- or -CH₂-, when Z is -O- and x indicates an integer of 4 to 16, y indicates an integer of 26 to 35, when Z is -O- and x indicates an integer of 17 to 25, y indicates an integer of 0 to 35, when Z is -CH₂- and x indicates an integer of 4 to 15, y indicates an integer of 26 to 35, when Z is -CH₂- and x indicates an integer of 16 to 25, y indicates an integer of 0 to 35) (hereinafter referred to as an "ASG (i.e., arthritis suppressor glycolipid)").

The ASG having the formula (I) induces slight proliferation of NKT cells and slight production of IFN-γ or other cytokines, but remarkably suppresses the second response of the prestimulated NKT cells (immunological unresponsiveness). An antibody arthritis model is reported to be mitigated the onset of arthritis in mice without NKT cells (J. Exp. Med. 2005. 201.41-7: Arthritis Rheum. 2005, 52, 1941), but the glycolipids described in the present invention strongly suppress the onset of that arthritis. Further, the ASG of formula (I) suppresses cellular infiltration mainly comprised of eosinophiles to the airway in the bronchial asthma animal model, suppresses the production of IL-5, IL-13 and other cytokines in alveolus washings, and suppresses airway sensitivity. That is, the inventors found that the ASG having the formula (I) is an effective glycolipid derivative which suppresses the inflammatory response induced by autoantibodies and can form a drug for treatment of autoimmune arthritis or other autoimmune diseases and bronchial asthma or other allergic diseases and, therefore, achieves the object of the present invention.

### BRIEF DESCRIPTION OF THE DRAWINGS

FIG. 1 is a graph showing the suppressive effects of synthesized glycolipid derivatives on K/BxN serum transferred arthritis (arthritis score).
FIG. 2 is a graph showing the suppressive effects of synthesized glycolipid derivatives on K/BxN serum transferred arthritis (pathological score).
FIG. 3 is a graph showing the suppressive effects of synthesized glycolipid derivatives on K/BxN serum transferred arthritis (arthritis score in absence of NKT cells).
FIG. 4 is a graph showing the effects of the present invention compounds on NKT cells.
FIG. 5 is a graph showing the effects on NKT cells of preadministration of the present invention compound.
FIG. 6 is a graph showing the suppression of cellular infiltration in alveolus washings in a bronchial asthma model by the present invention compound.
FIG. 7 is a graph showing the suppression of cytokine level in alveolus washings in a bronchial asthma model by the present invention compound.
FIG. 8 is a graph showing the suppressive effect of the present invention compound on airway resistance in a bronchial asthma model.
FIG. 9 is a graph showing the suppression of cellular infiltration in alveolus washings in a bronchial asthma model by the present invention compound.
FIG. 10 is a graph showing the suppression of cytokine level in alveolus washings in a bronchial asthma model by the present invention compound.
FIG. 11 is a graph showing the suppressive effects on airway resistance in alveolus washings in a bronchial asthma model by the present invention compound.

### BEST MODE FOR CARRYING OUT THE INVENTION

In the present invention, as the autoimmune disease, for example multiple sclerosis, myasthenia gravis, chronic rheumatoid arthritis, systemic lupus erythematosus, Sjogren syndrome, systemic scleroderma, polymyositis, dermatomyositis, insulin-dependent diabetes, idiopathic thrombocytopenic purpura, Hashimoto's thyroiditis, Basedow's disease, pernicious anemia, Addison's disease, atrophic gastritis, hemolytic anemia, Crohn's disease, ulcerative colitis, autoimmune hepatitis, pemphigus, pemphigoid, vasculitis syndrome, autoimmune hemolytic anemia, Goodpasture's syndrome, Lupus nephritis, etc. may be mentioned. Further, as the allergic diseases in the present invention, for example, bronchial asthma, atopic asthma, atopic dermatitis, allergic rhinitus, urticarial eruption, hayfever, drug allergy, contact dermatitis, organ transplant rejection, etc. may be mentioned.

The compounds having the formula (I) according to the present invention include the compounds having the general formulas (Ia), (Ib), (Ic) and (Id). 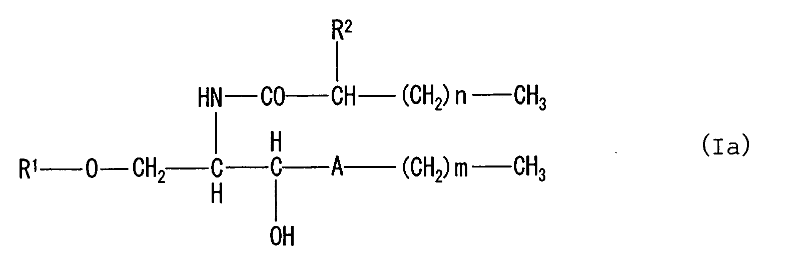 wherein m indicates an integer of 17 to 25, n indicates an integer of 0 to 35, R¹, R² and A are the same as defined above.

In the formula (Ia), as preferable examples of the aldopyranose residue shown by R¹, α-D-glucosyl, α-D-galactosyl, α-D-mannosyl, β-D-glucosyl, β-D-galactosyl, β-D-mannosyl, 2-deoxy-2-amino-α-D-galactosyl, 2-deoxy-2-amino-β-D-galactosyl, 2-deoxy-2-acetylamino-α-D-galactosyl, 2-deoxy-2-acetylamino-β-D-galactosyl, β-D-allopyranosyl, β-D-altropyranosyl, β-D-idosyl, etc. may be mentioned, particularly preferably α-D-glucosyl, α-D-galactosyl, α-D-mannosyl, 2-deoxy-2-amino-α-D-galactosyl, 2-deoxy-2-acetylamino-α-D-galactosyl, or other α-isomers. R² indicates a hydrogen atom or hydroxyl group, but is preferably a hydrogen atom. A indicates -CH₂-, -CH(OH)-CH₂-, or -CH=CHCH₂-, preferably -CH₂- or -CH(OH)-CH₂-, particularly preferably -CH(OH)-CH₂-. m indicates an integer of 17 to 25, preferably an integer of 17 to 22. n indicates an integer of 0 to 35, but preferably is an integer of 15 to 35, more preferably 18 to 31, most preferably 20 to 28. 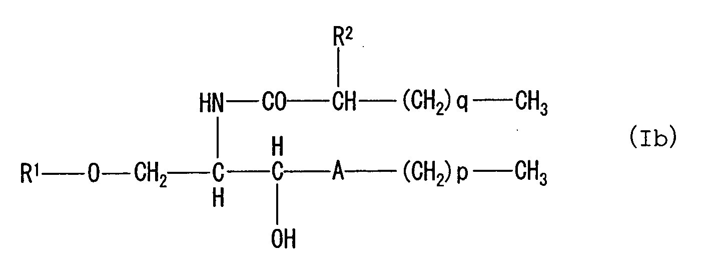 wherein p indicates an integer of 4 to 16, q indicates an integer of 26 to 35, ad R¹, R² and A are the same as defined above.

In the formula (Ib), as preferred examples of the aldopyranose residue shown in R¹, α-D-glucosyl, α-D-galactosyl, α-D-mannosyl, β-D-glucosyl, β-D-galactosyl, β-D-mannosyl, 2-deoxy-2-amino-α-D-galactosyl, 2-deoxy-2-amino-β-D-galactosyl, 2-deoxy-2-acetylamino-α-D-galactosyl, 2-deoxy-2-acetylamino-β-D-galactosyl, P-D-allopyranosyl, β-D-altropyranosyl, β-D-idosyl, etc. may be mentioned, particularly preferably α-D-glucosyl, α-D-galactosyl, α-D-mannosyl, 2-deoxy-2-amino-α-D-galactosyl, 2-deoxy-2-acetylamino-α-D-galactosyl, or other -α isomers. R² indicates a hydrogen atom or hydroxyl group, but is preferably a hydrogen atom. A indicates -CH₂-, -CH(OH)-CH₂-, or -CH=CHCH₂-, but is preferably -CH₂- or -CH(OH)-CH₂-, particularly preferably -CH(OH)-CH₂-. p indicates an integer of 4 to 16 and is preferably an integer of 12 to 16. q indicates an integer of 26 to 35 and is preferably an integer of 26 to 32. 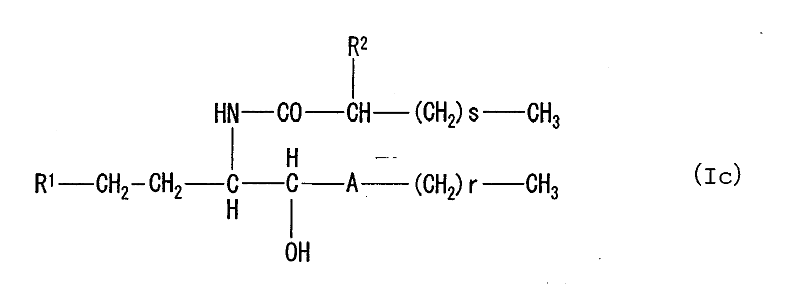 wherein r indicates an integer of 16 to 25, and s indicates an integer of 0 to 35, R¹, R² and A are the same as defined above.

In the formula (Ic), as preferred examples of the aldopyranose residue shown in R¹, α-D-glucosyl, α-D-galactosyl, α-D-mannosyl, β-D-glucosyl, β-D-galactosyl, β-D-mannosyl, 2-deoxy-2-amino-α-D-galactosyl, 2-deoxy-2-amino-β-D-galactosyl, 2-deoxy-2-acetylamino-α-D-galactosyl, 2-deoxy-2-acetylamino-β-D-galactosyl, β-D-allopyranosyl, β-D-altropyranosyl, β-D-idosyl, etc. may be mentioned, particularly preferably α-D-glucosyl, α-D-galactosyl, α-D-mannosyl, 2-deoxy-2-amino-α-D-galactosyl, 2-deoxy-2-acetylamino-α-D-galactosyl, and other α-isomers. R² indicates a hydrogen atom or hydroxyl group, preferably a hydrogen atom. A indicates -CH₂-, -CH(OH)-CH₂-, or -CH=CHCH₂-, preferably -CH₂- or -CH(OH)-CH₂-, particularly preferably -CH(OH)-CH₂-. r indicates an integer of 16 to 25, preferably an integer of 16 to 22. s indicates an integer of 0 to 35, preferably an integer of 15 to 35, more preferably 18 to 31, most preferably 20 to 28. 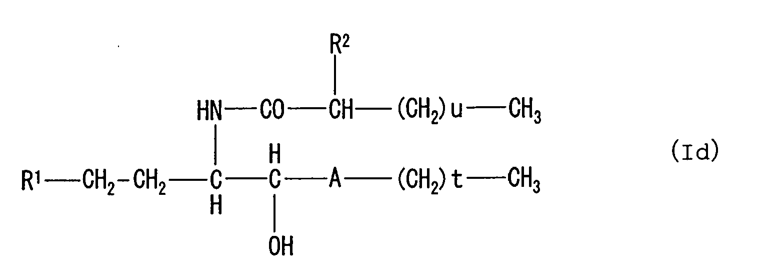 wherein t indicates an integer of 4 to 15, u indicates an integer of 26 to 35, and R¹, R² and A are the same as defined above.

In the formula (Id), as preferred examples of the aldopyranose residue shown in R¹, α-D-glucosyl, α-D-galactosyl, α-D-mannosyl, β-D-glucosyl, β-D-galactosyl, β-D-mannosyl, 2-deoxy-2-amino-α-D-galactosyl, 2-deoxy-2-amino-β-D-galactosyl, 2-deoxy-2-acetylamino-α-D-galactosyl, 2-deoxy-2-acetylamino-β-D-galactosyl, P-D-allopyranosyl, β-D-altropyranosyl, β-D-idosyl, etc. may be mentioned, particularly preferably α-D-glucosyl, α-D-galactosyl, α-D-mannosyl, 2-deoxy-2-amino-α-D-galactosyl, 2-deoxy-2-acetylamino-α-D-galactosyl, and other α-substituents. R² indicates a hydrogen atom or hydroxyl group, but is preferably a hydrogen atom. A indicates a -CH₂-, -CH(OH)-CH₂- or -CH=CHCH₂-, but is preferably -CH₂- or -CH(OH)-CH₂-, particularly preferably -CH(OH)-CH₂-. t indicates an integer of 4 to 15, preferably an integer of 12 to 15. u indicates an integer of 26 to 35, preferably an integer of 26 to 32.

Among the compounds having the formula (I), the particularly preferred examples are listed below: Namely, 2-hexacosanoylamino-1-O-α-D-galactopyranosyl-D-ribo-1,3,4-tricosanetriol, 2-nonacosanoylamino-1-O-α-D-galactopyranosyl-D-ribo-1,3,4-tricosanetriol, 2-hexacosanoylamino-1-O-α-D-galactopyranosyl-D-ribo-1,3,4-pentacosanetriol, 2-hexacosanoylamino-1-O-α-D-galactopyranosyl-D-ribo-1,3,4-heptacosanetriol, 2-triacontanoyl amino-1-O-α-D-galactopyranosyl-D-ribo-1,3,4-octadecanetriol, 2-tetratriacontanoyl amino-1-O-α-D-galactopyranosyl-D-ribo-1,3,4-octadecanetriol, 2-tetracosanoylamino-1-O-α-D-galactopyranosyl-D-ribo-1,3,4-tricosanetriol, 2-hexacosanoylamino-1-O-α-D-galactopyranosyl-D-ribo-1,3,4-tetracosanetriol, 2-nonacosanoylamino-1-O-α-D-galactopyranosyl-D-ribo-1,3,4-tetracosanetriol, 2-nonacosanoylamino-1-O-α-D-galactopyranosyl-D-ribo-1,3,4-pentacosanetriol, 2-nonacosanoylamino-1-O-α-D-galactopyranosyl-D-ribo-1,3,4-docosanetriol, 2-triacontanoyl amino-1-O-α-D-galactopyranosyl-D-ribo-1,3,4-nonadecanetriol, 2-triacontanoyl amino-1-O-α-D-galactopyranosyl-D-ribo-1,3,4-icosanetriol, 2-triacontanoyl amino-1-O-α-D-galactopyranosyl-D-ribo-1,3,4-henicosanetriol, 2-triacontanoyl amino-1-O-α-D-galactopyranosyl-D-ribo-1,3,4-docosanetriol, 2-pentacosanoylamino-1-O-α-D-galactopyranosyl-D-ribo-1,3,4-tricosanetriol, 2-heptacosanoylamino-1-O-α-D-galactopyranosyl-D-ribo-1,3,4-tricosanetriol, 2-octacosanoylamino-1-O-α-D-galactopyranosyl-D-ribo-1,3,4-tricosanetriol, (3S,4S,5R)-1-α-D-galactopyranosyl-3-hexacosanoyl amino-4,5-tetracosanediol, etc. may be mentioned.

The glycolipid derivatives having the formula (I) may be synthesized by various methods. For example, in the case of the derivatives (Ia) and (Ib), where Z is -O-, they may be synthesized according to the methods described below. These methods will now be successively explained.

First, the compound (VIa) is obtained from a known starting substance (IVa)(WO2004/072091; K. Murata et al., J. Org. Chem. 2005, 70, 2398). Further, a known method (e.g., M. Morita et al., J. Med. Chem. 1995, 38, 2176) is followed to obtain the compounds (IVb) and (IVc), then the double bond of the compound (IVb) is reduced to convert the compound to the compound (VIb) (step 1). From the compound (VIa), (VIb) or (IVc), the compound (VIIa), (VIIb) or (VIIc) is obtained (step 2), then selective reduction of the azide groups and an amidation reaction are used to obtain the compound (VIIIa), (VIIIb) or (VIIIc) (step 3). A glycosylation reaction of the compound (VIIa), (VIIb) or (VIIc) and compound (IX) is then used to obtain the compound (Xa), (Xb) or (Xc) (step 4). Selective reduction of the azide groups and an amidation reaction of-the compound (Xa), (Xb) or (Xc) are used to obtain the compound (XIa), (XIb) or (XIc). Further, the compound (XIa), (XIb) or (XIc) can be obtained by a glycosilylation reaction of the compound (VIIIa), (VIIIb) or (VIIIc) and the compound (IX) (step 5). Finally, the protective groups of the compound (XIa), (XIb) or (XIc) can be removed to obtain the desired compound (Ia) or (Ib) (step 6).

### Step 1

It is possible to synthesize the compound (VIa) from the known starting material (IVa). Further, it is possible to obtain the compounds (IVb) and (IVc) by a known method. (IVb) can be converted to the compound (VIb). 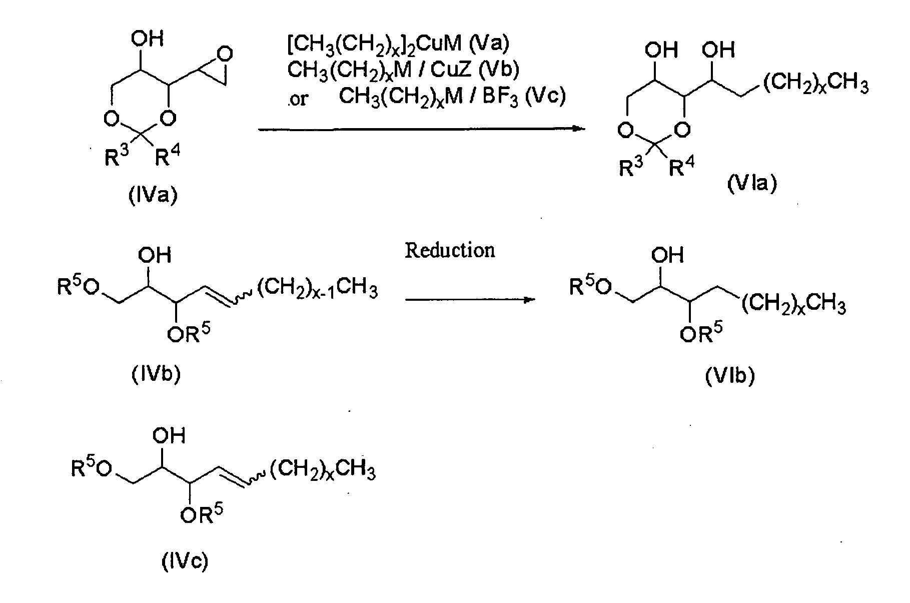 wherein x is as defined above, R³ and R⁴ may be the same or different and indicate a hydrogen atom, an alkyl group substituted or unsubstituted with a methyl group, ethyl group, isopropyl group, methoxy group, ethoxy group, trifluoromethyl group, chlorine atom, fluorine atom, etc., an aryl group substituted or unsubstituted with a methyl group, ethyl group, isopropyl group, fluorine atom, chlorine atom, bromine atom, iodine atom, nitro group, methoxy group, methoxymethyl group, trifluoromethyl group, etc., aralkyl group substituted or unsubstituted with a methyl group, ethyl group, isopropyl group, fluorine atom, chlorine atom, bromine atom, iodine atom, nitro group, methoxy group, methoxymethyl group, trifluoromethyl group, etc. or R³ and R⁴ together bond to form a cyclic structure of a propylene group, butylene group, or pentylene group, R⁵ indicates a benzyl group, p-methoxybenzyl group, p-nitrobenzyl group, p-methoxymethyloxybenzyl group, p-benzyloxybenzyl group, 3,4-dimethoxybenzyl group, diphenylmethyl group, or di(p-nitrophenyl)methyl group, M indicates Li, MgCl, MgBr, or MgI, and Z indicates a chlorine atom, bromine atom, or iodine atom).

In the step from the compound (IVa) to compound (VIa), in diethyl ether, tetrahydrofuran, dioxane, toluene, xylene, hexane, cyclohexane, or another inert solvent or a mixed solvent of the same containing copper (I) iodide, copper (I) bromide, copper (I) chloride, or borofluoride, 1 to 6 equivalents of alkyllithium reagent or a Grignard reagent was added to the compound (IVa) at -78°C to 0°C, preferably -50 to -10°C, the mixture was stirred at the same temperature, for example, for 1 to 5 hours, the compound (IVa) was added thereto and, then, the mixture was further stirred for 1 to 5 hours to obtain the desired compound (VIa). Further, in the step from the compound (IVb) to compound (VIb), the compound (IVb) may be stirred in diethyl ether, dimethoxyethane, tetrahydrofuran, dioxane, toluene, xylene, ethyl acetate, chloroform, dichloromethane, methanol, water, ethanol, isopropyl alcohol, or another solvent or the mixture thereof, if necessary, in the presence of sodium acetate, potassium acetate, sodium carbonate, sodium hydrogen carbonate, or another base, together with hydrazine or tosylhydrazine at 30 to 150°C or is hydrogenated in the presence of Pd-C, Pd-CaCO₃-Pb, Pd-BaSO₄, PtO₂, etc. at room temperature so as to convert the compound (VIb) to the compound (IVb).

The compound obtained by this reaction can be used directly as a starting material for the next step, but, if necessary, can also be utilized after purification by a generally used purification method, for example, recrystallization or column chromatography.

### Step 2

It is possible to convert the compound (VIa) obtained at step 1 to the compound (VIIa) or (VIIb). Further, it is possible to convert the compound (VIb) or (IVc) obtained at step 1 to the compound (VIIc). 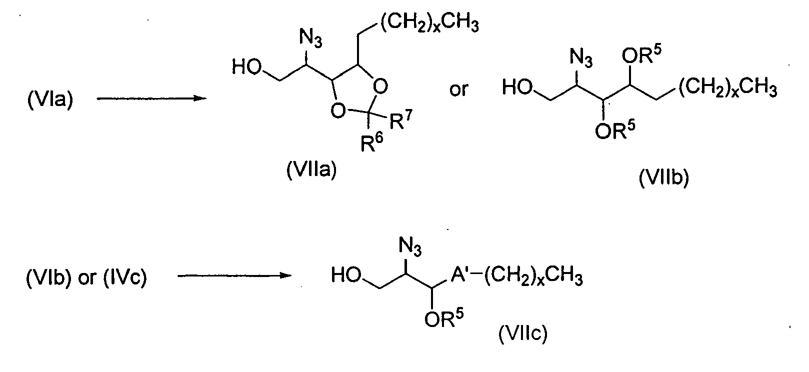 wherein x and R⁵ are the same as defined above, R⁶ and R⁷ may be the same or different and indicates a hydrogen atom, an alkyl group substituted or unsubstituted by a methyl group, ethyl group, isopropyl group, methoxy group, ethoxy group, trifluoromethyl group, chlorine atom, fluorine atom, etc., an aryl group substituted or unsubstituted by a methyl group, ethyl group, isopropyl group, fluorine atom, chlorine atom, bromine atom, iodine atom, nitro group, methoxy group, methoxymethyl group, trifluoromethyl group, etc., or an aralkyl group substituted or unsubstituted with a methyl group, ethyl group, isopropyl group, fluorine atom, chlorine atom, bromine atom, iodine atom, nitro group, methoxy group, methoxymethyl group, trifluoromethyl group, etc. or R⁶ and R⁷ together bond to form a cyclic structure of a propylene group, butylene group, or pentylene group and A' indicates -CH₂- or -CH=CHCH₂-.

The compound (VIa) may be reacted in methylene chloride, 1,2-dichloroethane, chloroform, carbon tetrachloride, acetonitrile, diethyl ether, tetrahydrofuran, dioxane, benzene, toluene, xylene, ethyl acetate, or another inert solvent in the presence of triethylamine, diisopropylethylamine, pyridine, sodium carbonate, sodium hydrogen carbonate, potassium carbonate, potassium hydrogen carbonate, or another base at -20°C to 100°C, preferably -10°C to 80°C with 1 to 5 equivalents of methanesulfonyl chloride, methanesulfonate anhydride, ethanesulfonyl chloride, 1-propanesulfonyl chloride, 1-butanesulfonyl chloride, trifluoromethanesulfonyl chloride, α-toluenesulfonyl chloride, benzenesulfonyl chloride, p-toluenesulfonyl chloride, p-toluenesulfonate anhydride, 4-methoxybenzenesulfonyl chloride, 4-chlorobenzenesulfonyl chloride, 2-nitrobenzenesulfonyl chloride, 3-nitrobenzenesulfonyl chloride, 4-nitrobenzenesulfonyl chloride, or another sulfonylation agent, for example, for 1 to 72 hours and deacetalated by an ordinary method. For the deacetalation conditions, the many methods described in "Protective Groups In Organic Synthesis" (John Wiley & Sons) etc. may be used. For example, the compound may be stirred in a mixture of hydrochloric acid, sulfuric acid, nitric acid, acetic acid, trifluoroacetic acid, methanesulfonic acid, trifluoromethanesulfonic acid, or another inorganic acid or organic acid and methanol, ethanol, 2-propanol, dioxane, methylene chloride, 1,2-dichloroethane, chloroform, carbon tetrachloride, benzene, toluene, xylene, water, or another inert solvent at -10°C to 100°C, preferably 0 to 50°C, so as to obtain the desired substance. Next, the compound thus obtained is reacted in acetonitrile, diethyl ether, tetrahydrofuran, dioxane, benzene, toluene, xylene, dimethyl sulfoxide, dimethyl formamide, or another inert solvent with 1 to 50 equivalents of sodium azide, lithium azide, tetra-n-butylammonium azide, etc. at 0 to 200°C, preferably 20 to 120°C. At this time, if necessary, triethylamine, diisopropylethylamine, pyridine, sodium carbonate, sodium hydrogen carbonate, potassium carbonate, potassium hydrogen carbonate, or another base may also be added. The compound obtained is acetalated to obtain the compound (VIIa). As the acetal conditions, the various methods described in "Protective Groups In Organic Synthesis" (John Wiley & Sons) etc. may be used. That is, the compound may be reacted in the presence of an organic acid or inorganic acid under solvent-less conditions or in diethyl ether, dioxane, benzene, toluene, xylene, or another inert solvent with an acetalation reagent at 0 to 200°C, preferably 20 to 120°C so as to obtain the desired compound (VIIa). At this time, as the acetalation reagent, acetone, 2,2-dimethoxypropane, 2-methoxypropene, 2-ethoxypropene, benzaldehyde, benzaldehyde dimethylacetal, cyclohexanone, cyclohexanone dimethylacetal, cyclopentanone, cyclopentanone dimethylacetal, etc. may be used. Further, the compound obtained after the azidization can be detritylated after tritylation or silylation of the primary hydroxyl groups and, then, arylmethylation of the other secondary hydroxyl groups so as to obtain the compound (VIIb). As the tritylation or silylation conditions, for example, the reaction conditions of 0.8 to 2 equivalents of trityl bromide, trityl chloride or trimethylsilyl chloride, t-butyl dimethylsilyl chloride, phenyl dimethylsilyl chloride, t-butyl dimethylsilyl trifluoromethanesulfonate in diethyl ether, tetrahydrofuran, dioxane, benzene, toluene, xylene, dimethyl formamide, dimethyl sulfoxide, or another inert solvent in the presence of lithium carbonate, potassium carbonate, sodium carbonate, sodium hydrogen carbonate, potassium hydrogen carbonate, lithium hydroxide, sodium hydroxide, potassium hydroxide, sodium hydroxide, potassium hydride, sodium, potassium, triethylamine, diisopropylethylamine, pyridine, lutidine or another base at -50°C to 120°C, preferably -20°C to 80°C may be mentioned. Further, as the arylmethylation agent, benzyl chloride, benzyl bromide, p-methoxybenzyl chloride, m-methoxybenzyl chloride, p-nitrobenzyl chloride, p-nitrobenzyl bromide, etc. may be mentioned. As the arylmethylation reaction conditions, the above tritylation conditions may be used. Further, as the conditions for detritylation, the various methods described in "Protective Groups In Organic Synthesis" (John Wiley & Sons) etc. may be used. For example, the conditions of reaction under solvent-less conditions or in methylene chloride, chloroform, 1,2-dichloroethane, tetrahydrofuran, acetonitrile, benzene, toluene, xylene, dioxane, water, methanol, ethanol, 2-propanol, tert-butanol, or another solvent in the presence of formic acid, acetic acid, trifluoroacetic acid, hydrochloric acid, sulfuric acid, nitric acid, or another acid or copper (II) sulfate at -50°C to 150°C, preferably -20°C to 100°C may be mentioned. As the desilylation conditions, in addition to the above detritylation conditions, the conditions of reaction in tetrahydrofuran, acetonitrile, methylene chloride, chloroform, 1,2-dichloroethane, or another solvent in the presence of tetrabutylammonium fluoride, potassium fluoride, hydrogen fluoride, hydrogen fluoride pyridine, etc. at -50°C to 150°C, preferably -20°C to 100°C may be mentioned.

The compound obtained by this reaction can be used directly as a starting material for the next step, but if ncessary, can also be utilized after purification by a generally used purification method, for example, recrystallization or column chromatography.

### Step 3

The azide groups of the compound (VIIa), (VIIb) and (VIIc) obtained at step 2 may be reduced to amino groups, then converted to amide groups to thereby obtain the compounds (VIIIa), (VIIIb) and (VIIIc). 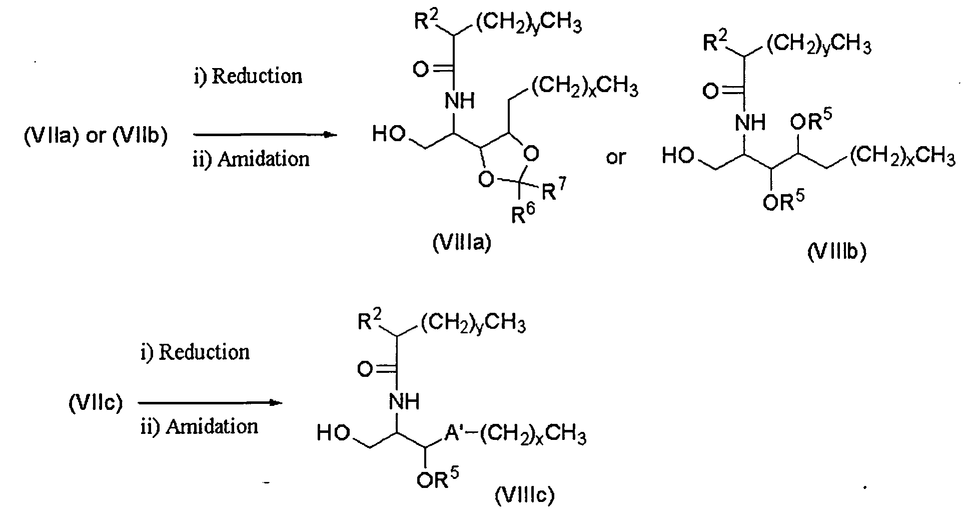 wherein R², R⁵, R⁶, R⁷, x, y, and A' are the same as defined above.

First, the compound (VIIa), (VIIb) or (VIIc) is treated with zinc/hydrochloric acid, lithium aluminum hydride or another metal reagent or triphenylphosphine, trimethylphosphine, triethyl phosphine, tributylphosphine or another triarylphosphine or trialkyl phosphine or is hydrogenated in the presence of Pd-C, Pd-CaCO₃-Pb, Pd-BaSO₄, PtO₂, etc. at room temperature to selectively reduce the azide groups to amino groups, then an amidation reaction with carboxylic acid is used to obtain the compound (VIIIa), (VIIIb) or (VIIIc). For the amidation reaction, the various methods described in "Compendium for Organic Synthesis" (Wiley-Interscience; A Division of John Wiley & Sons) etc. may be utilized. As one example, by reacting an amine with a corresponding carboxylic acid, in an inert solvent such as methylene chloride, chloroform, 1,2-dichloroethane, diethyl ether, tetrahydrofuran, dioxane, acetonitrile, benzene, toluene, xylene, dimethyl formamide, in the presence of an activation agent of carboxylic acid at -50°C to 120°C, preferably -20°C to 80°C, the desired compound (VIIIa), (VIIIb) or (VIIIc) can be obtained. As the activation reagent of carboxylic acid, silicon tetrachloride, acetic anhydride, acetyl chloride, ethyl chlorocarbonate, 2-iodo-1-methylpyridinium iodide, 2-chloro-1-methylpyridinium iodide, diphenylphosphinyl chloride, N,N'-dicyclohexylcarbodiimide (DCC), N-hydroxybenzotriazole/DCC, 1-ethyl-3-(3-diethylaminopropyl)carbodiimide hydrochloride, ethoxyacetylene, trimethylsilylethoxyacetylene, carbodiimidazole, diphenylphosphoryl azide, diethylphosphoryl cyanidate, etc. may be mentioned. Further, if necessary, p-toluenesulfonic acid, polyphosphoric acid or another acid, or triethylamine, diisopropylethylamine, N-methylmorpholine, pyridine, 2,6-lutidine, 4-dimethylaminopyridine or another base may be added.

The compound obtained by the above reaction can be used directly as a starting material for the next step, but if necessary, can also be utilized after purification by a generally used purification method, for example, recrystallization or column chromatography.

### Step 4

The glycosidation reaction of the compound (VIIa), (VIIb) or (VIIc) and the compound (IX) obtained at step 2 can be used to obtain the compound (Xa), (Xb) or (Xc). 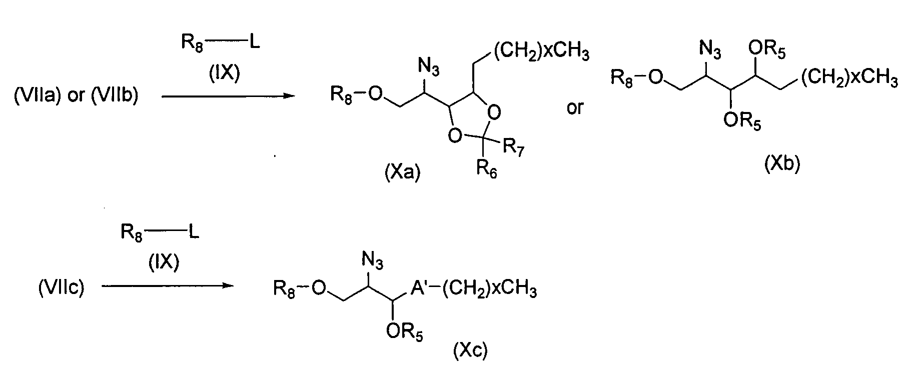 wherein R⁵, R⁶, R⁷, x and A' are the same as defined above, R⁸ indicates an aldopyranosyl group protected with a hydroxyl group or amino group, and L indicates a chlorine atom, bromine atom, fluorine atom or iodine atom.

The compound (VIIa), (VIIb) or (VIIc) can be reacted with the compound (IX) in an inert solvent such as hexane, cyclohexane, methylene chloride, chloroform, 1,2-dichloroethane, ether, tetrahydrofuran, acetonitrile, benzene, toluene, xylene, dioxane, dimethyl formamide, dichloromethane or the mixed solvent thereof in the presence of borotrifluoride, silver perchlorate, stannous (II) chloride, titanium tetrachloride, stannous tetrachloride or another Lewis acid or in the presence of tetra-n-butylammonium bromide or another halogenated ammonium salt, for example, at -100°C to 50°C, preferably -78°C to 30°C, to obtain the compound (Xa), (Xb) or (Xc). The Lewis acid or halogenated ammonium salt used in this reaction may be used alone or in any combinations thereof. Further, at that time, if necessary, a molecular sieve may be added.

Further, as the aldopyranose residue protected on hydroxyl or amino groups forming R⁸, 2,3,4,6-tetra-O-benzyl-D-glucosyl, 2,3,4,6-tetra-O-benzyl-D-galactosyl, 2,3,4,6-tetra-O-benzyl-D-mannosyl, 3,4,6-tri-O-benzyl-2-deoxy-2-(tert-butoxycarbonyl amino)-D-galactosyl, 3,4,6-tri-O-benzyl-2-deoxy-2-acetylamino-D-galactosyl, 2,3,4,6-tetra-O-benzyl-D-allopyranosyl, 2,3,4,6-tetra-O-benzyl-β-D-altropyranosyl, 2,3,4,6-tetra-O-benzyl-β-D-idosyl, 3,4,6-tri-O-benzyl-2-deoxy-2-(dibenzylamino)-D-galactosyl, etc. may be mentioned.

The compound (Xa), (Xb) or (Xc) obtained by the above reaction can be used directly as a starting material for the next step, but, if necessary, can also be utilized after purification by a generally used purification method, for example, recrystallization or column chromatography.

### Step 5

By reducing the azide group of the compound (Xa), (Xb) or (Xc) obtained at step 4 to an amino group, then converting it to an amide group, it is possible to obtain a compound (XIa), (XIb) or (XIc). Further, a glycosidation reaction of the compound (VIIIa), (VIIIb) or (VIIIc) obtained at step 3 and the compound (IX) can be used to obtain the compound (XIa), (XIb) or (XIc). 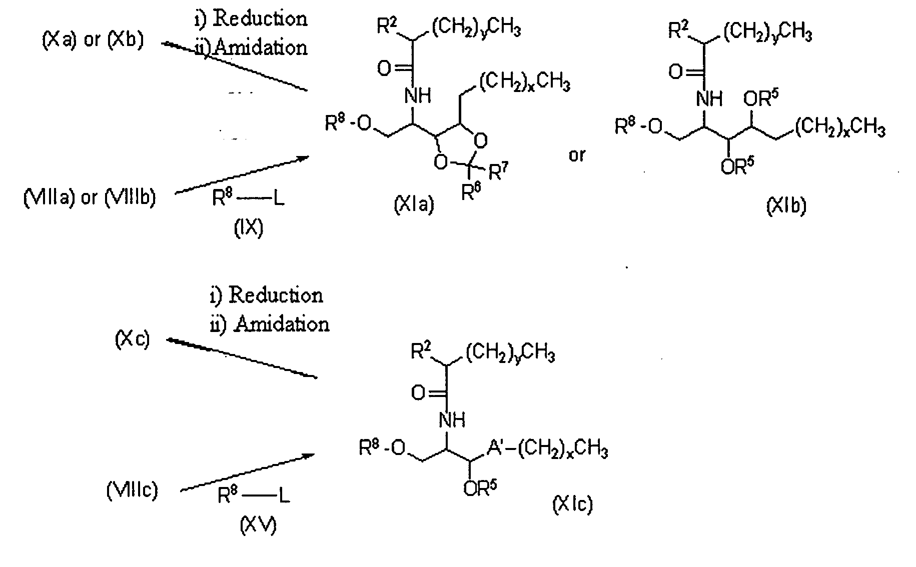 wherein R², R⁵, R⁶, R⁷, R⁸, x, y, and A' are the same as defined above.

The compound (Xa), (Xb) or (Xc) can be converted to the compound (XIa), (XIb) or (XIc) by the same method as in step 3. Further, the compound (VIIIa), (VIIIb) or (VIIIc) can be converted to the compound (XIa), (XIb) or (XIc) by the same method as in step 4.

The compound obtained by the above reaction can be used directly as a material for the next step, but, if necessary, can also be utilized after purification by a generally used purification method, for example, recrystallization or column chromatography.

### Step 6

The compound (XIa) obtained at step 5 can be deacetalated and dearylmethylated or the compound (XIb) or (XIc) can be dearylmethylated to obtain the compound (Ia) or (Ib). 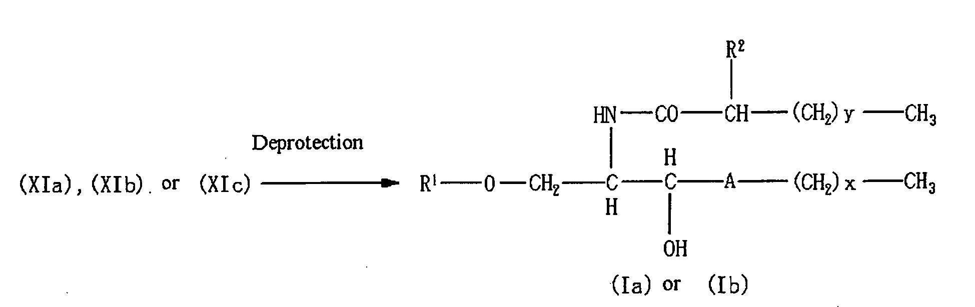 wherein R¹, R², R⁵, R⁶, R⁷, R⁸, x, y, A, and A' are the same as defined above.

As the deacetalation and dearylmethylation conditions, the various methods described in "Protective Groups In Organic Synthesis" (John Wiley & Sons) etc. may be used. For example, as the deacetalation conditions, the method shown in step 2 may be used. Further, as the dearylmethylation conditions, the conditions of adding 4-methylcyclohexene in methanol, ethanol, 2-propanol, ethyl acetate, tetrahydrofuran, dimethyl formamide or another solvent not participating in the reaction in the presence of Pd-C, Pd(OH)₂, PtO₂, etc. for heating and reflux or hydrogenation at room temperature may be mentioned.

The compound obtained by the reaction may, if nescessary, be purified by a generally used purification method, for example, recrystallization or column chromatography.

In the glycolipid derivatives of the general formula (I), the derivatives (Ic) and (Id), where Z is -CH₂-, may, for example, be synthesized by the methods shown below. These methods will now be successively explained, but the synthesis method is not limited to these methods.

Among the glycolipid derivatives of the formula (I), where Z is -CH₂-, that is, the derivatives (Ic) and (Id), those where A is -CH₂- or -CH(OH)CH₂- may be synthesized from a known starting substance (XIIa) (e.g., Sabino, A. A. et al., Tetrahedron Lett. 2002, 43, 2819; Toba, T. et al., Tetrahedron Lett. 2005, 46, 5043), a known starting substance (XIIb) (e.g., Bestmann, H. J. et al., Angew. Chem. 1991, 103, 78), or a known starting substance (XIIc) (e.g., Chen, X. et al., Tetrahedron Lett. 2002, 43, 3529). First, (XIIa), (XIIb) or (XIIc) is converted to the compound (XIVa), (XIVb) or (XIVc) respectively (step 7), then oxidized to obtain the compound (XVa), (XVb) or (XVc) (step 8). The compound (XVa), (XVb) or (XVc) and a known ethynyl aldopyranose derivative (XVI) (e.g., Dondoni, A. et al., J. Org. Chem. 2002, 67, 4475; Toba, T. et al., Tetrahedron Lett. 2005, 46, 5043) are reacted to obtain the compound (XVIIa), (XVIIb) or (XVIIc) (step 9), the compound (XVIIIa), (XVIIIb) or (XVIIIc) is derived (step 10), then the azide group is selectively reduced to obtain an amine which is then condensed with carboxylic acid (XIX) to obtain the compound (XXa), (XXb) or (XXc) (step 11). All of the protective groups of the compound (XXa), (XXb) or (XXc) are removed to obtain the desired compound (Ic) or (Id) (step 12).

Further, among the glycolipid derivatives of the formula (I), where Z is -CH₂-, that is, the derivatives (Ic) and (Id), those where A is -CH=CH₂CH₂- may be synthesized as follows: After a reaction with a known starting substance (XXI) (Toyota, M. et al., Heterocycles 1995, 40, 115) and a known starting substance (XVI) (e.g., Dondoni, A. et al., J. Org. Chem. 2002, 67, 4475; Toba, T. et al., Tetrahedron Lett. 2005, 46, 5043), then reduction of the unsaturated bonds, the compound (XXII) is obtained (step 13). The hydroxyl groups are converted to azide groups to obtain the compound (XXIII) (step 14). The protection is removed from the compound (XXIII), the primary hydroxyl group is selectively protected to obtain the compound (XXIV) (step 15), then the azide groups are selectively reduced to obtain an amine body; which is then condensed with the carboxylic acid (XIX) to obtain the compound (XXV) (step 16). The hydroxyl groups of the compound (XXV) are protected, then only the protective groups of the primary hydroxyl groups are selectively removed to convert the compound to the compound (XXVI) (step 17), then the primary hydroxyl groups are oxidized, then the carbon is increased to obtain the compound (XXVIII) (step 18). All of the protective groups of compound (XXVIII) are removed to obtain the targeted compound (Ic) or (Id) (step 19).

### Step 7:

From the known starting substance (XIIa) (e.g., Sabino, A. A. et al., Tetrahedron Lett. 2002, 43, 2819; Toba, T. et al., Tetrahedron Lett. 2005, 46, 5043) or known starting substance (XIIb) (e.g., Bestmann, H. J. et al., Angew. Chem. 1991, 103, 78), or known starting substance (XIIc) (e.g., Chen, X. et al., Tetrahedron Lett. 2002, 43, 3529), the compounds of formulae (XIVa), (XIVb) and (XIVc) can be synthesized. 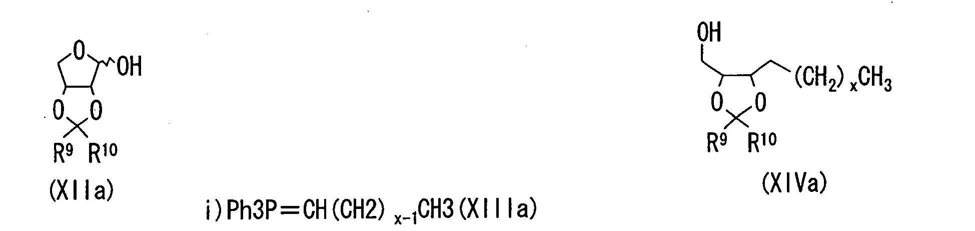 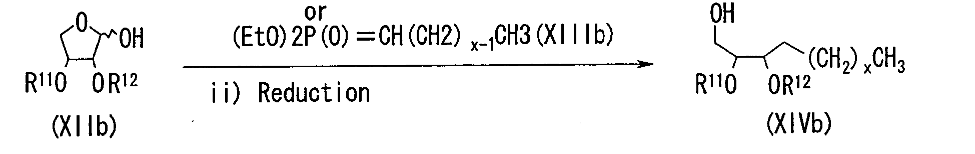 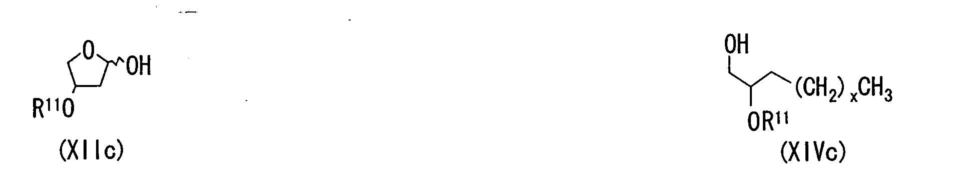 wherein x is the same as defined above, R⁹ and R¹⁰ may be the same or different, a hydrogen atom, a substituted or unsubstituted alkyl group, a substituted or unsubstituted aryl group, a substituted or unsubstituted aralkyl group, or R⁹ and R¹⁰ may together bond to form a cyclic structure of a propylene group, butylene group or pentylene group, R¹¹ and R¹² may be the same or different, benzyl group, p-methoxybenzyl group, p-nitrobenzyl group, p-methoxymethyloxybenzyl group, p-benzyloxybenzyl group, 3,4-dimethoxybenzyl group, diphenylmethyl group, di(p-nitrophenyl)methyl group, methoxymethyl group, benzyloxymethyl group, p-methoxybenzyloxy group, nitrobenzyloxy group, 2,2,2-trichloroethoxymethyl group, tetrahydropyranyl group.

By adding sodium, potassium, sodium hydride, potassium hydride, n-butyllithium, lithium diisopropylamide, bis(trimethylsilyl)amide potassium, bis(trimethylsilyl)amide lithium or another base to the corresponding phosphonium salt or alkylphosphoric acid ester in tetrahydrofuran, ether, toluene, hexamethylphosphoric amide or another solvent not participating in the reaction or the mixed solvents thereof at -78 to 50°C, preferably -78 to 0°C, to prepare a phosphoylide compound (XIIIa) or (XIIIb) and reacting the reaction product under the same conditions with the compound (XIIa), (XIIb) or (XIIc), the unsaturated body of desired compound (XIVa), (XIVb) or (XIVc) can be obtained.

By reducing the unsaturated compound, the desired compound (XIVa), (XIVb) or (XIVc) can be obtained. As the reduction conditions of the double bonds, the various methods described in "Shinjikken Kagaku Koza" (New Experimental Chemistry Seminar) (Maruzen) etc. may be used. For example, the conditions of adding 4-methylcyclohexene in methanol, ethanol, 2-propanol, ethyl acetate, tetrahydrofuran, dimethyl formamide or another solvent not participating in the reaction in the presence of Pd-C, Pd(OH)₂, PtO₂, Rh-C, Ru-C or another transition metal catalyst for heating and reduction or hydrogenation at room temperature may be mentioned.

The compound (XIVa), (XIVb) or (XIVc) obtained by the above method can be used directly as a starting material for producing the compound (XVa), (XVb) or (XVc), but, if necessary, can also be utilized after purification by a generally used purification method, for example, recrystallization or column chromatography.

### Step 8:

The compound (XIVa), (XIVb) or (XIVc) obtained at step 7 may be oxidized to obtain the compound (XVa), (XVb) or (XVc). 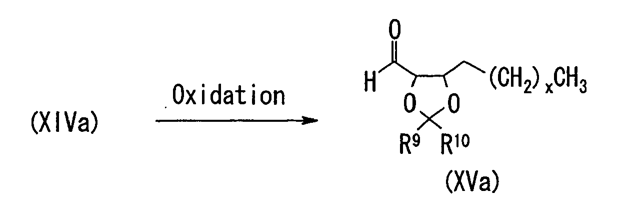 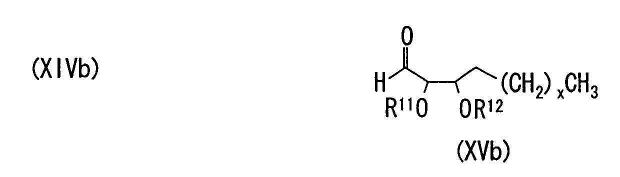 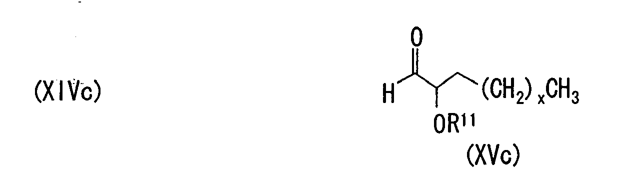 wherein x, R⁹, R¹⁰, R¹¹ and R¹² are the same as defined above.

For the oxidation reaction of the primary hydroxyl groups to aldehyde groups, the various methods described in the "Compendium for Organic Synthesis)" (Wiley Interscience; A division of John Wiley & Sons) etc. may be utilized. For example, by reacting the compound (XIVa), (XIVb) or (XIVc) in tetrahydrofuran, dioxane, acetonitrile, dichloromethane, chloroform, dimethyl formamide, pyridine or another solvent not participating in the reaction or the mixed solvents thereof at room temperature to 50°C with 1 to 20 equivalents, preferably 1 to 5 equivalents, of Dess-Martin reagent [1,1,1-tris(acetyloxy)-1,1-dehydro-1,2-benziodoxole-3-(1H)-one] (D. B. Dess and J. C. Martin, J. Am. Chem. Soc. 1991, 113, 7277), the compounds (XVa), (XVb) and (XVc) can be obtained.

The compound (XVa), (XVb) and (XVc) obtained from the above reaction can be used directly as a starting material for the next step, but, if necessary, can also be utilized after purification by a generally used purification method, for example, recrystallization or column chromatography.

### Step 9:

The compounds (XVa), (XVb) and (XVc) obtained at step 8 can be reacted with a known ethynyl aldopyranose derivative (XVI) (e.g., Dondoni, A. et al., J. Org. Chem. 2002, 67, 4475; Toba, T. et al., Tetrahedron Lett. 2005, 46, 5043), then the unsaturated bonds reduced to obtain the compounds (XVIIa), (XVIIb) and (XVIIc). 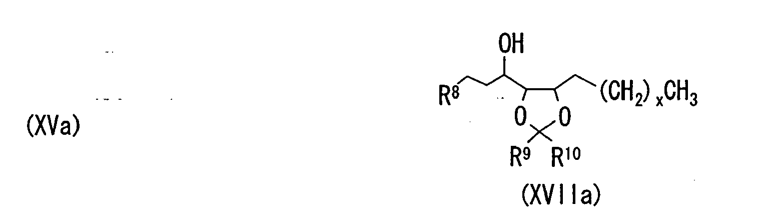 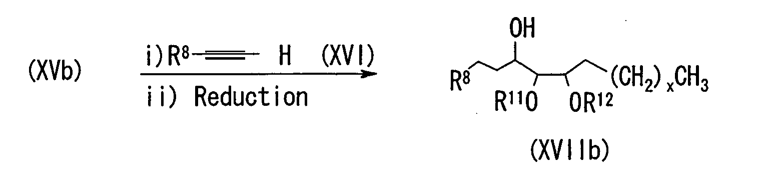 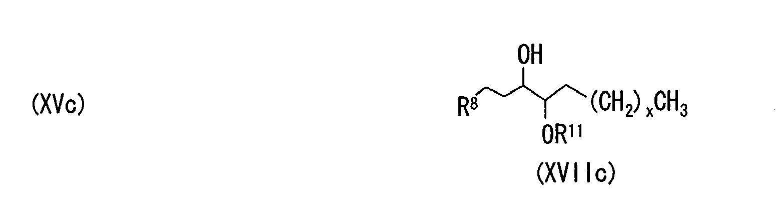 wherein x, R⁸, R⁹, R¹⁰, R¹¹ and R¹² are the same as defined above.

The addition reaction of aldehyde and terminal alkyne derivative can be performed by various methods, but, for example, a terminal alkyne derivative may be treated in methylene chloride, 1,2-dichloroethane, carbon tetrachloride, diethyl ether, tetrahydrofuran, dioxane, benzene, toluene, xylene or another inert solvent not participating in the reaction at -78 to 150°C, preferably -78 to 50°C, by 1 to 2 equivalents of triethylamine, diisopropylethylamine, pyridine, lutidine, 1,8-diazabicyclo[5,4,0]-7-undecene (DBU), or another organic base or sodium carbonate, sodium hydrogen carbonate, potassium carbonate, potassium hydrogen carbonate, cesium carbonate, cesium fluoride, sodium, potassium, sodium hydride, potassium hydride, sodium methoxide, sodium ethylate, potassium tert-butoxide, n-butyllithium, lithium diisopropylamide, bis(trimethylsilyl)amide potassium, bis(trimethylsilyl)amide lithium or another inorganic base, then reacting the reaction product, with aldehyde at -78 to 150°C, preferably -78 to 50°C, more preferably -78 to -20°C.

The reduction of the unsaturated bonds may be performed under the conditions shown in step 7 and also under the conditions using the diimide produced by the oxidation of hydrazine, the decomposition of azodicarboxylic acid salt, the decomposition in the decomposition system of N-sulfonyl hydrazide in excess as a reducing agent and reacting the same in 1,2-dichloroethane, 1,2-dimethoxyethane, carbon tetrachloride, diethyl ether, tetrahydrofuran, dioxane, benzene, toluene, xylene, acetonitrile or another inert solvent not participating in the reaction at -40 to 150°C, preferably 50 to 100°C.

The compounds (XVIIa), (XVIIb) and (XVIIc) obtained by the above reaction can be used directly as a starting material for the next step, but, if necessary, can also be utilized after purification by a generally used purification method, for example, recrystallization or column chromatography.

### Step 10:

The compound (XVIIa), (XVIIb) or (XVIIc) obtained at step 9 can be used to obtain the compound (XVIIIa), (XVIIIb) or (XVIIIc). 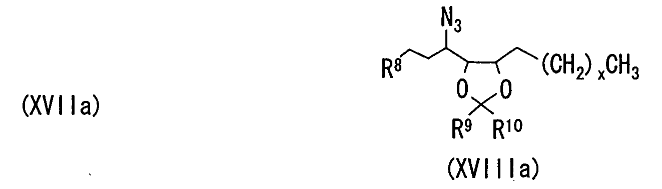 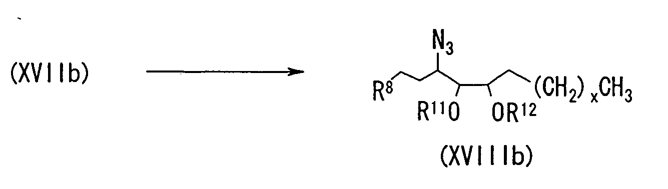 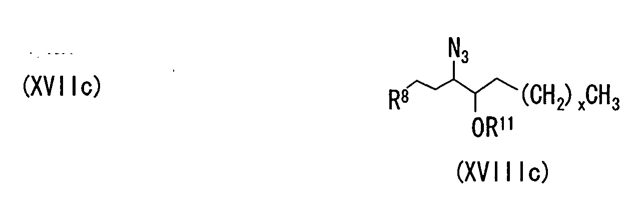 wherein x, R⁸, R⁹, R¹⁰, R¹¹ and R¹² are the same as defined above.

The compound (XVIIa), (XVIIb) or (XVIIc) is reacted with 1 to 5 equivalents of methanesulfonyl chloride, methanesulfonate anhydride, ethanesulfonyl chloride, 1-propanesulfonyl chloride, 1-butanesulfonyl chloride, trifluoromethanesulfonyl chloride, α-toluenesulfonyl chloride, benzenesulfonyl chloride, p-toluenesulfonyl chloride, p-toluenesulfonate anhydride, 4-methoxybenzenesulfonyl chloride, 4-chlorobenzenesulfonyl chloride, 2-nitrobenzenesulfonyl chloride, 3-nitrobenzenesulfonyl chloride, 4-nitrobenzenesulfonyl chloride or another sulfonylation agent in methylene chloride, 1,2-dichloroethane, chloroform, carbon tetrachloride, acetonitrile, diethyl ether, tetrahydrofuran, dioxane, benzene, toluene, xylene, ethyl acetate, dimethyl formamide or another inert solvent in the presence of triethylamine, diisopropylethylamine, pyridine, sodium carbonate, sodium hydrogen carbonate, potassium carbonate, potassium hydrogen carbonate or another base at -20 to 100°C, preferably -10 to 80°C, for 1 hour to 72 hours and an ordinary method is used to form a sulfonic acid ester. Then, the compound obtained is reacted in acetonitrile, diethyl ether, tetrahydrofuran, dioxane, benzene, toluene, xylene, dimethyl sulfoxide, dimethyl formamide or another inert solvent with 1 to 50 equivalents of sodium azide lithium azide, tetrabutylammonium azide or another azidation agent at 0 to 200°C, preferably 20 to 120°C. At this time, if necessary, triethylamine, diisopropylethylamine, pyridine, sodium carbonate, sodium hydrogen carbonate, potassium carbonate, potassium hydrogen carbonate or another base may be added.

The compounds (XVIIIa), (XVIIIb) and (XVIIIc) obtained by the above reaction can be used directly as a starting material for the next step, but as needed can also be utilized after purification by a generally used purification method, for example, recrystallization or column chromatography.

### Step 11:

The azide group of the compound (XVIIIa), (XVIIIb) or (XVIIIc) obtained at step 10 can be reduced to an amino group, then amidated to form the compound (XXa), (XXb) or (XXc). 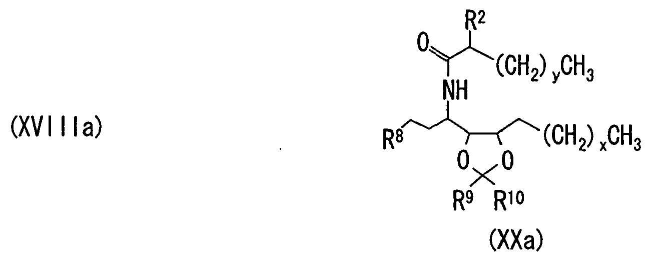 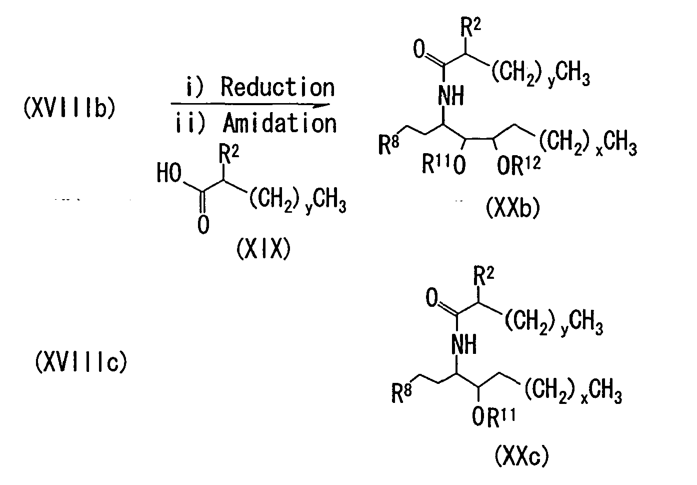 wherein x, y, R², R⁸, R⁹, R¹⁰, R¹¹, and R¹² are the same as defined above.

First, the compound (XVIIIa), (XVIIIb) or (XVIIIc) is treated with zinc/hydrochloric acid, lithium aluminum hydride or another metal reagent or triphenylphosphine, trimethylphosphine, triethylphosphine, tributylphosphine or another triarylphosphine or trialkyl phosphine or is hydrogenated in the presence of Pd-C, Pd-CaCO₃-Pb, Pd-BaSO₄, PtO₂, etc. at room temperature so as to selectively reduce the azide group to an amino group, then an amidation reaction with carboxylic acid is used to derive the compound (XXa), (XXb) or (XXc). For the amidation reaction, the various methods described in the "Compendium for Organic Synthesis" (Wiley-Interscience; A Division of John Wiley & Sons) etc. may be utilized. As one example, an amine compound may be reacted with a corresponding carboxylic acid so as to obtain the targeted compound (XXa), (XXb) or (XXc) in methylene chloride, chloroform, 1,2-dichloroethane, diethyl ether, tetrahydrofuran, dioxane, acetonitrile, benzene, toluene, xylene, dimethyl formamide or another inert solvent in the presence of an activation agent of carboxylic acid at -50 to 120°C, preferably -20 to 80°C. As the activation reagent for the carboxylic acid, silicon tetrachloride, acetic anhydride, acetyl chloride, ethyl chlorocarbonate, 2-iodo-1-methyl pyridinium iodide, 2-chloro-1-methyl pyridinium iodide, diphenylphosphinyl chloride, N,N'-dicyclohexylcarbodiimide(DCC), N-hydroxybenzotriazole/DCC, 1-ethyl-3-(3-diethylaminopropyl)carbodiimide hydrochloride, ethoxyacetylene, trimethylsilylethoxyacetylene, carbodiimidazole, diphenylphosphoryl azide, diethylphosphoryl cyanidate etc. may be mentioned. Further, if necessary, p-toluenesulfonic acid, polyphosphoric acid or another acid or triethylamine, diisopropylethylamine, N-methyl morpholine, pyridine, 2,6-lutidine, dimethylaminopyridine or another base, N-hydroxybenzotriazole, 1-hydroxy-7-azabenzotriazole or other activating agent may also be added.

The compound obtained by the above reaction can be used directly as a starting material for the next step, but, if necessary, can also be utilized after purification by a generally used purification method, for example, recrystallization or column chromatography.

### Step 12:

The compound (XXa), (XXb) or (XXc) obtained at step 11 may be deacetalated and dearylmethylated to obtain the compound (Ic) or (Id). 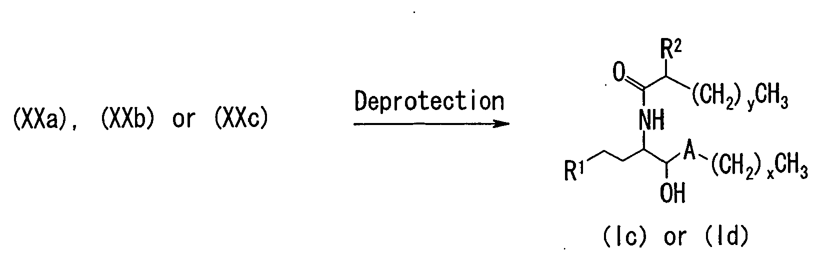 wherein A indicates -CH₂-or -CH (OH) CH₂-, and x, y, R¹, R², R⁸, R⁹, R¹⁰, R¹¹ and R¹² are the same as defined above.

As the deprotection conditions, the various methods described in "Protective Groups In Organic Synthesis" (John Wiley & Sons) etc. may be used. For example, as the deacetalation conditions, the method of adding, in a mixed solution of methanol, ethanol, 2-propanol, dioxane, methylene chloride, 1,2-dichloroethane, chloroform, carbon tetrachloride, benzene, toluene, xylene or another inert solvent, to hydrochloric acid, sulfuric acid, nitric acid, acetic acid or another inorganic acid, trifluoroacetic acid, methanesulfonic acid, trifluoromethanesulfonic acid or another organic acid or inert solvent, stannous chloride, aluminum chloride, dimethyl bromoborane, trifluoroborane diethyl ether complex, trimethylsilyl iodide or another Lewis acid and, if necessary, thiophenol or another soft nucleating agent and stirring at -10 to 100°C, preferably 0 to 50°C may be mentioned. Further, as the dearylmethylation conditions, the conditions of adding in methanol, ethanol, 2-propanol, ethyl acetate, tetrahydrofuran, dimethyl formamide or another solvent not participating in the reaction, in the presence of Pd-C, Pd(OH)₂, PtO₂ Rh-C, Ru-C or another transition metal catalyst, 4-methylcyclohexene and heating and refluxing the same or hydrogenation at room temperature and further the method of stirring, in such an inert solvent, stannous chloride, aluminum chloride trifluoroborane diethyl ether complex, trimethylsilyl iodide or another Lewis acid, the method of stirring in an inert solvent together with DDQ, CAN, or another oxidizing agent, the method of electrical oxidation, etc. may be mentioned.

The compound obtained by the above reaction may be, if necessary, purified by a generally used purification method, for example, recrystallization or column chromatography.

### Step 13:

A reaction of a known starting substance (XXI) (Toyota, M. et al., Heterocycles 1995, 40, 115) and known starting substance (XVI) (e.g., Toba, T. et al., Tetrahedron Lett. 2005, 46, 5043) followed by reduction may be used to obtain the compound (XXII). 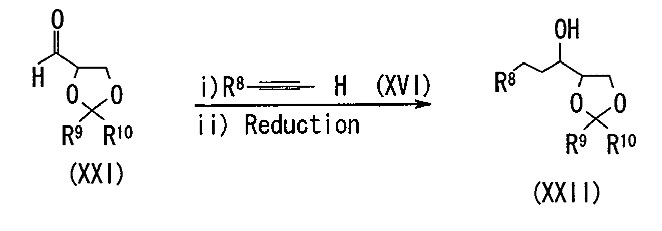 wherein R⁸, R⁹ and R¹⁰ are the same as defined above.

As the conditions for the addition reaction and reduction of the unsaturated bonds, the method shown in step 9 may be used.

The compound (XXII) obtained by the above reaction can be used directly as a material for the next step, but, if necessary, can also be utilized after purification by a generally used purification method, for example, recrystallization or column chromatography.

### Step 14:

The compound (XXII) obtained at step 13 can be converted to the compound (XXIII). 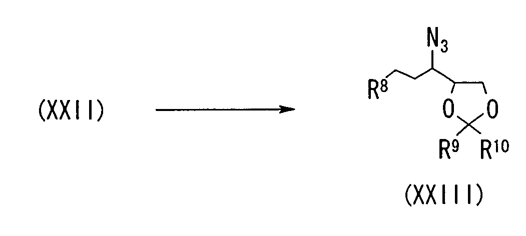 wherein R⁸, R⁹ and R¹⁰ are the same as defined above.

The derivation of the sulfonic acid ester and azidation reaction may be performed by the method shown in step 10.

The compound (XXIII) obtained by the above reaction can be used directly as a starting material for the next step, but, if necessary, can also be utilized after purification by a generally used purification method, for example, recrystallization or column chromatography.

### Step 15:

After removing the protection, the compound (XXIII) obtained at step 14, the primary hydroxyl groups can be selectively protected to obtain the compound (XXIV). 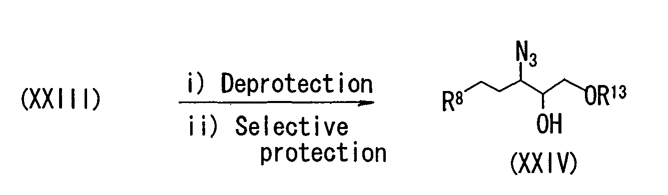 wherein R⁸, R⁹ and R¹⁰ are the same as defined above, R¹³ is a substituted or unsubstituted alkyl group, substituted or unsubstituted aralkyl group, substituted or unsubstituted silyl group, substituted or unsubstituted acyl group, substituted or unsubstituted alkoxycarbonyl group or substituted or unsubstituted alkylaminocarbonyl group.

As the deacetalation conditions, the method shown in step 12 may be used. As the method for selectively protecting the primary hydroxyl group, the protective groups are introduced at a low temperature, preferably -78°C to -20°C, utilizing the difference in reactivities, the catalytic amount to 1.2 equivalents of di-n-butyltin oxide are added to improve the selectivity of the primary hydroxyl groups or trityl chloride, t-butyldimethylsilyl chloride, t-butyldiphenylsilyl triflate or another steric hindrance protective agent may be used in an amount of 1 to 1.5 equivalents. For example, as the t-butyldimethylsilylation conditions, the conditions of reacting 0.8 to 2 equivalents of t-butyldimethylsilyl chloride or t-butyldimethylsilyl triflate in diethyl ether, tetrahydrofuran, dioxane, benzene, toluene, xylene, dimethyl formamide, dimethyl sulfoxide or another inert solvent in the presence of lithium carbonate, potassium carbonate, sodium carbonate, sodium hydrogen carbonate, potassium hydrogen carbonate, lithium hydroxide, sodium hydroxide, potassium hydroxide, sodium hydride, potassium hydride, sodium, potassium, triethylamine, iisopropylethylamine, pyridine, lutidine or another base at -50 to 120°C, preferably -20 to 80°C, may be mentioned.

The compound (XXIV) obtained by the above reaction can be used directly as a starting material for the next step, but as needed can also be utilized after purification by a generally used purification method, for example, recrystallization or column chromatography.

### Step 16:

The azide group of the compound (XXIV) obtained at step 15 may be selectively reduced to obtain an amine compound and then condensed with the carboxylic acid (XIX) to obtain the compound (XXV). 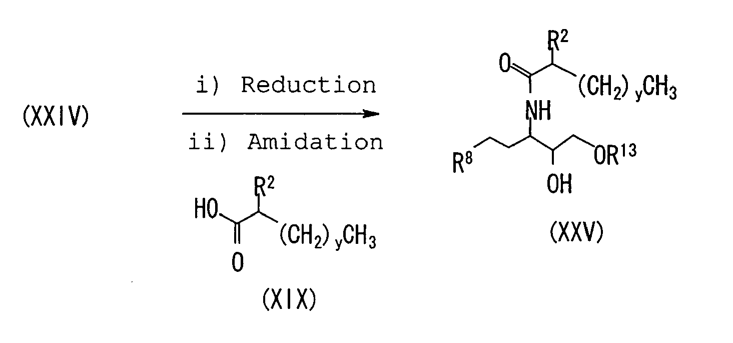 wherein y, R², R⁸ and R¹³ are the same as defined above.

The reduction of the azide group and the condensation reaction with the carboxylic acid may be performed by the method shown in step 11.

The compound (XXV) obtained by the above reaction can be used directly as a starting material for the next step, but as needed can also be utilized after purification by a generally used purification method, for example, recrystallization or column chromatography.

### Step 17:

The hydroxyl group of the compound (XXV) obtained at step 16 can be protected, then only the protective group of the primary hydroxyl group selectively removed to convert the compound to the compound (XXVI). 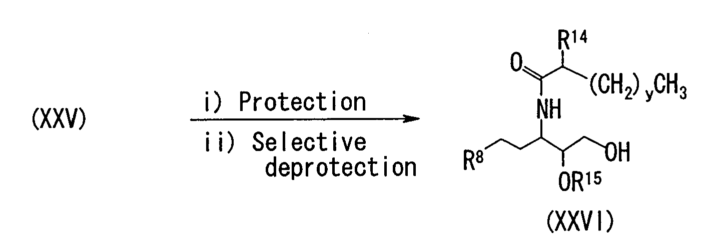 wherein y, R², R⁸ and R¹³ are the same as defined above, R¹⁴ indicates hydrogen or OR¹⁵, R¹⁵ indicates a benzyl group, p-methoxybenzyl group, p-nitrobenzyl group, p-methoxymethyloxybenzyl group, p-benzyloxybenzyl group, 3,4-dimethoxybenzyl group, diphenylmethyl group, di(p-nitrophenyl)methyl group, methoxymethyl group, benzyloxymethyl group, p-methoxybenzyloxy group, nitrobenzyloxy group, 2,2,2-trichloroethoxymethyl group or tetrahydropyranyl group.

As the protection of the hydroxyl group, for example, the conditions of reacting 0.8 to 5 equivalents of an arylmethylation agent in diethyl ether, tetrahydrofuran, dioxane, benzene, toluene, xylene, dimethyl formamide, dimethyl sulfoxide or another inert solvent in the presence of lithium carbonate, potassium carbonate, sodium carbonate, sodium hydrogen carbonate, potassium hydrogen carbonate, lithium hydroxide, sodium hydroxide, potassium hydroxide, sodium hydride, potassium hydride, sodium, potassium, triethylamine, diisopropylethylamine, pyridine, lutidine or another base at -50 to 120°C, preferably -20 to 80°C, may be mentioned. As the arylmethylation agent, benzyl chloride, benzyl bromide, p-methoxybenzyl chloride, m-methoxybenzyl chloride, p-nitrobenzyl chloride, p-nitrobenzyl bromide, etc. may be mentioned. Alternatively, as the alkoxymethylation method, the conditions of reacting alkoxymethyl chloride or alkoxymethyl bromide under similar conditions to said arylmethylation or stirring a dialkoxymethane in diethyl ether, dioxane, benzene, toluene, xylene, dimethyl formamide, dimethyl sulfoxide or another inert solvent in the presence of hydrochloric acid, sulfuric acid, nitric acid, acetic acid, trifluoroacetic acid, methanesulfonic acid, trifluoromethanesulfonic acid or another inorganic acid or organic acid or trimethylsilyl trifluoromethanesulfonate or another Lewis acid catalyst at -10 to 100°C, preferably 0 to 50°C may be mentioned.

In the case of selective removal of the protective groups of the primary hydroxyl groups, for example, in the case of substituted silyl groups, it is possible to effect the selective removal by reacting the protective groups with tetra-n-butylammonium fluoride, HF-pyridine complex salt, HF-KF, potassium fluoride or other desilylation agent having fluoride ions in pentane, hexane, heptane, cyclohexane, benzene, toluene, xylene, diethyl ether, diisopropyl ether, t-butylmethyl ether, cyclopentylmethyl ether, tetrahydrofuran, dioxane, 1,2-dimethoxyethane, dichloromethane, chloroform, carbon tetrachloride, 1,2-dichloroethane, trichloroethylene, acetonitrile, ethyl acetate, dimethyl formamide, dimethyl sulfoxide, or another inert solvent at -10 to 100°C, preferably 0 to 50°C, so as to selectively remove them as distinguished from other protective groups. Further, acetal-based protective groups or protective groups where t-butyl groups are bonded with heteroatoms may be removed distinguished from the protective groups of aralkyl-based hydroxyl groups by reacting with hydrochloric acid, sulfuric acid, nitric acid, acetic acid, trifluoroacetic acid, methanesulfonic acid, trifluoromethane sulfonic acid or another inorganic acid or organic acid at -20 to 100°C, preferably 0 to 50°C. Further, alkylcarbonyl- or alkoxycarbonyl-based protective groups may be selectively removed distinguished from silyl-based, acetal-based and alkyl-based protective groups by reacting with sodium hydroxide, lithium hydroxide, potassium hydroxide, calcium hydroxide, barium hydroxide, sodium carbonate, lithium carbonate, potassium carbonate and another inorganic base in aqueous solution or sodium methoxide, sodium ethoxide, potassium ethoxide or another metal alkoxide at -20 to 100°C, preferably 0 to 50°C.

The compound (XXVI) obtained by the above reaction can be used directly as a starting material for the next step, but, if necessary, can also be utilized after purification by a generally used purification method, for example, recrystallization or column chromatography.

### Step 18:

The primary hydroxyl group of the compound (XXVI) obtained at step 17 may be oxidized, then (XXVIII) derived. 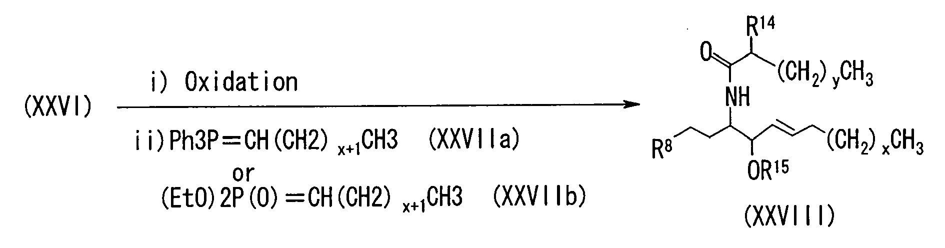 wherein x, y, R⁸, R¹⁴ and R¹⁵ are the same as defined above.

The oxidation of the primary hydroxyl group may be performed by the method shown in step 8. Further, the carbonation reaction from the aldehyde to an unsaturated compound may be performed by the method shown in step 7.

The compound (XXVIII) obtained by the above reaction can be used directly as a starting material for the next step, but, if necessary, can also be utilized after purification by a generally used purification method, for example, recrystallization or column chromatography.

### Step 19:

The compound (XXVIII) obtained at step 18 may be deacetalated and dearylmethylated to obtain the compound (Ic) or (Id). 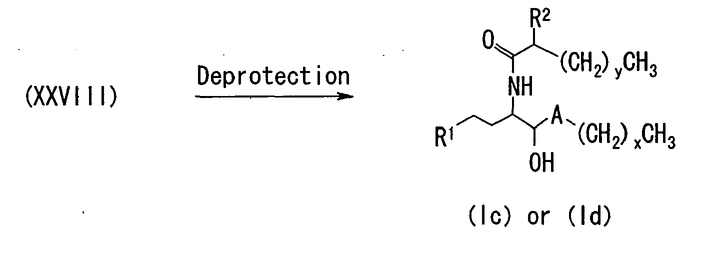 wherein A indicates -CH=CH₂CH₂- and x, y, R¹, R², R⁸, R¹⁴ and R¹⁵ are as defined above.

As the deprotection conditions, the method shown in step 6 may be used.

The compound obtained by the reaction may, if necessary, be purified by a generally used purification method, for example, recrystallization or column chromatography.

The compound having the formula (I) of the present invention may be administered alone or may be as desired prepared together with another ordinary pharmacologically acceptable known commonly used vehicle into a preparation aimed at the improvement or treatment of an autoimmune disease, allergic disease or disease where it is known that NKT cells or stimulus of NKT cells is participating in the deterioration of disease conditions. For example, the active ingredient may be administered alone, or together with, a commonly used excipient as a capsule, tablet, injection or other suitable form orally or parenterally. For example, capsules are prepared by mixing the powder material with lactose, starch or its derivative, a cellulose derivative or another excipient and packing the mixture in gelatin capsules. Further, in addition to the excipient, carboxymethylcellulose sodium, alginic acid, gum Arabic or another binder and water are added and kneaded, the mixture is granulated, if necessary, then talc, stearic acid or another lubricante further added and a usual tablet press is used for preparation. At the time of parenteral administration by injection, the active ingredient may be dissolved together with a solubility aid in sterilized distilled water or sterilized physiological saline and sealed in ampoules for preparation of injections. If necessary, a stabilizer or buffer may be included.

The dosage of the drug for improvement or treatment of autoimmune diseases, allergic diseases or diseases, in which NKT cells or stimulus to NKT cells is known to be participating in the deterioration of the disease conditions of the present invention depends on various factors such as the symptoms, age, route of administration, form of drug, number of dosages, etc. of the patient to be treated, but usually 0.001 mg to 5000 mg/day/person, preferably 0.01 mg to 500 mg/day/person, more preferably 0.1 mg to 100 mg/day/person is suitable.

### EXAMPLES

Examples will now be used to more specifically explain the present invention, but the scope of the present invention is of course not limited to these

### Examples.

### Example 1: Synthesis of 1,3-O-benzylidene-D-arabino-1,2,3,4-tricosanetetraol (Compound 1)

To a suspension of copper (II) iodide (2.14 g, 11.2 mmol) in dehydrated tetrahydrofuran (50 ml), 1.58M n-octadecyl magnesium bromide (49.5 mmol in tetrahydrofuran solution) was added dropwise at -40°C, then the mixture was stirred at -10°C for 10 minutes. Next, a dehydrated tetrahydrofuran (100 ml) solution of known 4,5-anhydro-1,3-O-benzylidene-D-arabitol (WO2004/072091; K. Murata et al., J. Org. Chem. 2005, 70, 2398) (5.01 g, 22.5 mmol) was added dropwise at -10°C, then the mixture was stirred at -10°C for 2.5 hours. To the reaction mixed solution, an aqueous saturated ammonium chloride solution was added, the product was extracted with ethyl acetate, the organic layer was washed with saturated saline, and the resultant mixture was dried over sodium sulfate, filtered, and concentrated in vacuo. The residue obtained was purified by silica gel column chromatography (chloroform:ethyl acetate=1:2) to obtain the above-identified compound in an amount of 3.05 g (yield 28%).

### Example 2: Synthesis of 1,3-O-benzylidene-2-O-methane sulfonyl-D-arabino-1,2,3,4-tricosanetetraol (Compound 2)

To a solution of the compound 1 synthesized in Example 1 (1.64 g, 3.44 mmol) in dehydrated pyridine (30 ml) and chloroform (30 ml), methanesulfonyl chloride (0.84 ml) was added dropwise at room temperature, then the reaction mixture was stirred at room temperature for 2 days through the night. The reaction mixture was concentrated, then heptane was used to azeotropically remove the solvent (twice), then the residue obtained was dissolved in chloroform (300 ml) and washed with water (twice). The organic layer was obtained, then dried over sodium sulfate, filtered, and concentrated in vacuo. The residue obtained was purified by silica gel column chromatography (chloroform:ethyl acetate=2:1) to obtain the above-identified compound in an amount of 1.00 g (yield 53%).

### Example 3: Synthesis of 2-O-methane sulfonyl-D-ribo-1,3,4-tricosanetriol (Compound 3)

To a solution of Compound 2 synthesized in Example 2 (555 mg, 1.00 mmol) in ethanol (50 ml) and chloroform (25 ml), 20% Pd(OH)₂/C (198 mg) was added, then the mixture was stirred under a hydrogen atmosphere at room temperature overnight. The catalyst was filtered off by serite, then the filtrate was concentrated in vacuo to obtain the above-identified compound in an amount of 445 mg (yield 95%).

### Example 4: Synthesis of 2-azido-D-ribo-1,2,3,4-tricosanetriol (Compound 4)

To a dehydrated dimethyl formamide (15 ml) solution of Compound 2 synthesized in Example 2 (416 mg, 0.89 mmol), sodium azide (237 mg) was added, then the mixture was stirred at 90°C for 6 hours. To the reaction mixture, ethyl acetate (400 ml) was added, then the resultant mixture was washed with water (twice), dried over sodium sulfate, filtered, then concentrated in vacuo. The residue obtained was purified by silica gel column chromatography (n-hexane:ethyl acetate=1:1) to obtain the above-identified compound in an amount of 149 mg (yield 41%).

### Example 5: Synthesis of 2-azido-3,4-O-isopropylidene-D-ribo-1,3,4-tricosanetriol (Compound 5)

To an acetone (20 ml) solution of Compound 4 synthesized in Example 4 (144 mg, 2.56 mmol), concentrated hydrochloric acid (20 µl) was added, then the mixture was stirred at room temperature for 1 hour. Next, the reaction mixture was neutralized with excess calcium hydroxide, the inorganic salt was removed by filtration through a cotton plug, then the resultant mixture was concentrated in vacuo. The residue obtained was purified by silica gel column chromatography (n-hexane:ethyl acetate=6:1) to obtain the above-identified compound in an amount of 76.7 mg (yield 48%).

### Example 6: Synthesis of 2-azido-1-O-(2,3,4,6-tetra-O-benzyl-α-D-galactopyranosyl)-3,4-O-isopropylidene-D-ribo-1,3,4-tricosanetriol (Compound 6)

To dried molecular sieve (4A, powder) (250 mg), a solution of toluene (4.5 ml) and dimethyl formamide (4.5 ml) of Compound 5 synthesized in Example 5 (100 mg, 0.28 mmol) and 2,3,4,6-tetra-O-benzyl-α-D-galactopyranosyl bromide (280 mg, 0.46 mmol) was added, then tetra-n-butylammonium bromide (218 mg, 0.68 mmol) was added and the resultant mixture was stirred over 4 days. To the reaction mixture, ethyl acetate (200 ml) was added, then the mixture was washed with an aqueous saturated sodium hydrogen carbonate solution and water. The organic layer was dried over sodium sulfate, filtered, and concentrated in vacuo, then the residue obtained was purified by silica gel column chromatography (n-hexane:ethyl acetate=25:1 to 8:1) to obtain the above-identified compound in an amount of 91.3 mg (yield 42%).

### Example 7: Synthesis of 2-amino-1-O-(2,3,4,6-tetra-O-benzyl-α-D-galactopyranosyl)-3,4-O-isopropylidene-D-ribo-1,3,4-tricosanetriol (Compound 7)

To the solution of Compound 6 synthesized in Example 6 (45 mg, 0.046 mmol) in ethanol (4.5 ml) and methylene chloride (1.5 ml), palladium-calcium carbonate (lead poisoned) (Lindlar's catalyst) (145 mg) was added, then the mixture was stirred under ordinary pressure at room temperature overnight for hydrogenation. The catalyst was filtered out, then the filtrate was concentrated in a vacuum to obtain the above-identified compound in an amount of 38.4 mg (yield 87%).

### Example 8: Synthesis of 2-hexacosanoylamino-1-O-(2,3,4,6-tetra-O-benzyl-α-D-galactopyranosyl)-3,4-O-isopropylidene-D-ribo-1,3,4-tricosanetriol (Compound 8)

To a mixed suspension of Compound 7 synthesized in Example 7 (38 mg, 0.04 mmol) in dimethyl formamide (3 ml) and methylene chloride (1.5 ml), n-hexacosanoic acid (18 mg, 0.046 mmol), and 1-hydroxyazabenzotriazole (7.8 mg, 0.057 mmol), triethylamine (13 µl, 0.093 mmol) and 1-ethyl-3-(3-dimethylaminopropyl)carbodiimide hydrochloride (14 mg, 0.073 mmol) were added under ice cooling, then the resultant mixture was stirred at room temperature for 5 days. The reaction mixture was diluted with ethyl acetate (100 ml), then was washed with an aqueous saturated sodium hydrogen carbonate solution and water, then the organic layer was dried over sodium sulfate, filtered, and concentrated in a vacuum. The residue obtained was purified by silica gel column chromatography (n-hexane:ethyl acetate=4:1) to obtain to obtain the above-identified compound in an amount of 51 mg (yield 96%) .

### Example 9: Synthesis of 2-hexacosanoylamino-1-O-(2,3,4,6-tetra-O-benzyl-α-D-galactopyranosyl)-D-ribo-1,3,4-tricosanetriol (Compound 9)

A methanol (0.8 ml)/methylene chloride (5 ml)/4N hydrochloric acid-dioxane (100 µl) mixed solution of Compound 8 synthesized in Example 8 (35 mg, 0.027 mmol) was stirred at room temperature for 2 hours, then concentrated in vacuo. The residue obtained was purified by silica gel column chromatography (hexane:ethyl acetate=2:1) to obtain the above-identified compound in an amount of 20 mg (yield 59%).

### Example 10: Synthesis of 2-hexacosanoylamino-1-O-α-D-galactopyranosyl-D-ribo-1,3,4-tricosanetriol (Compound 10)

To a solution of Compound 9 synthesized in Example 9 (20 mg, 0.016 mmol) in methanol (3 ml)/methylene chloride (1.5 ml), palladium hydroxide (9.8 mg) was added and the mixture hydrogenated by stirring at ordinary pressure and room temperature for 4 hours. The catalyst was filtered out and the filtrate concentrated in a vacuum to obtain the above-identified compound in an amount of 13 mg (yield 92%).

### Example 11: Synthesis of 2-nonacosanoylamino-1-O-α-D-galactopyranosyl-D-ribo-1,3,4-tricosanetriol (Compound 11)

From the compound 7 synthesized in Example 7 and n-nonacosanoic acid, the same experimental procedure as in Examples 8 to 10 was carried out to obtain the above-identified compound.

### Example 12: Synthesis of 2-hexacosanoylamino-1-O-α-D-galactopyranosyl-D-ribo-1,3,4-pentacosanetriol (Compound 12)

From known 4,5-anhydro-1,3-O-benzylidene-D-arabitol and n-icosyl magnesium bromide, the same experimental procedure as in Examples 1 to 7 was used to obtain 2-amino-1-O-(2,3,4,6-tetra-O-benzyl-α-D-galactopyranosyl)-3,4-O-isopropylidene-D-ribo-1,3,4-pentacosanetriol. From the resultant compound and n-hexacosanoic acid, the same experimental procedure as in Examples 8 to 10 was carried out to obtain the above-identified compound.

### Example 13: Synthesis of 2-hexacosanoylamino-1-O-α-D-galactopyranosyl-D-ribo-1,3,4-heptacosanetriol (Compound 13)

From known 4,5-anhydro-1,3-O-benzylidene-D-arabitol and n-docosyl magnesium bromide, the same experimental procedure as in Examples 1 to 7 was used to obtain 2-amino-1-O-(2,3,4,6-tetra-O-benzyl-α-D-galactopyranosyl)-3,4-O-isopropylidene-D-ribo-1,3,4-heptacosanetriol. From this and n-hexacosanoic acid, the same experimental procedure as in Examples 8 to 10 was carried out to obtain the above-identified compound.

### Example 14: Synthesis of 2-triacontanoyl amino-1-O-α-D-galactopyranosyl-D-ribo-1,3,4-octadecanetriol (Compound 14)

From known 4,5-anhydro-1,3-O-benzylidene-D-arabitol and n-tridecyl magnesium bromide, the same experimental procedure as in Examples 1 to 7 was used to obtain 2-amino-1-O-(2,3,4,6-tetra-O-benzyl-α-D-galactopyranosyl)-3,4-O-isopropylidene-D-ribo-1,3,4-octadecanetriol. From the resultant compound and n-triacontanoic acid, the same experimental procedure as in Examples 8 to 10 was carried out to obtain the above-identified compound.

### Example 15: Synthesis of 2-tetratriacontanoyl amino-1-O-α-D-galactopyranosyl-D-ribo-1,3,4-octadecanetriol (Compound 15)

From known 4,5-anhydro-1,3-O-benzylidene-D-arabitol and n-tridecyl magnesium bromide, the same experimental procedure as in Examples 1 to 7 was used to obtain 2-amino-1-O-(2,3,4,6-tetra-O-benzyl-α-D-galactopyranosyl)-3,4-O-isopropylidene-D-ribo-1,3,4-octadecanetriol. From the resultant compound and n-tetratriacontanoic acid, the same experimental procedure as in Examples 8 to 10 was carried out to obtain the above-identified compound.

### Example 16: Synthesis of 2-tetracosanoylamino-1-O-α-D-galactopyranosyl-D-ribo-1,3,4-tricosanetriol (Compound 16)

From Compound 7 synthesized in Example 7 and n-tetracosanoic acid, the same experimental procedure as in Examples 8 to 10 was performed to obtain the above-identified compound.

### Example 17: Synthesis of 2-hexacosanoylamino-1-O-α-D-galactopyranosyl-D-ribo-1,3,4-tetracosanetriol (Compound 17)

From known 4,5-anhydro-1,3-O-benzylidene-D-arabitol and n-nonadecyl magnesium bromide, the same experimental procedure as in Examples 1 to 7 was used to obtain 2-amino-1-O-(2,3,4,6-tetra-O-benzyl-α-D-galactopyranosyl)-3,4-O-isopropylidene-D-ribo-1,3,4-tetracosanetriol. From the resultant compound and n-hexacosanoic acid, the same experimental procedure as in Examples 8 to 10 was carried out to obtain the above-identified compound.

### Example 18: Synthesis of 2-nonacosanoylamino-1-O-α-D-galactopyranosyl-D-ribo-1,3,4-tetracosanetriol (Compound 18)

From known 4,5-anhydro-1,3-O-benzylidene-D-arabitol and n-nonadecyl magnesium bromide, the same experimental procedure as in Examples 1 to 7 was used to obtain 2-amino-1-O-(2,3,4,6-tetra-O-benzyl-α-D-galactopyranosyl)-3,4-O-isopropylidene-D-ribo-1,3,4-tetracosanetriol. From the resultant compound and n-nonacosanoic acid, the same experimental procedure as in Examples 8 to 10 was carried out to obtain the above-identified compound.

### Example 19: Synthesis of 2-pentacosanoylamino-1-O-α-D-galactopyranosyl-D-ribo-1,3,4-tricosanetriol (Compound 19)

From Compound 7 synthesized at Example 7 and n-pentacosanoic acid, the same experimental procedure as in Examples 8 to 10 was carried out to obtain the above-identified compound.

### Example 20: Synthesis of 2-heptacosanoylamino-1-O-α-D-galactopyranosyl-D-ribo-1,3,4-tetracosanetriol (Compound 20)

From Compound 7 synthesized at Example 7 and n-heptacosanoic acid, the same experimental procedure as in Examples 8 to 10 was carried out to obtain the above-identified compound.

### Example 21: Synthesis of 2-octacosanoylamino-1-O-α-D-galactopyranosyl-D-ribo-1,3,4-tetracosanetriol (Compound 21)

From Compound 7 synthesized at Example 7 and n-octacosanoic acid, the same experimental procedure as in Examples 8 to 10 was carried out to obtain the above-identified compound.

### Example 22: Synthesis of 4S,5R-2,3-O-isopropylidene-1,2,3-docosanetriol (Compound 22)

To a THF (30 ml) suspension of phosphonium salt (5.97 g, 10.0 mmol) prepared from bromooctadecane and triphenylphosphine, 1.6M n-butyllithium hexane solution (6.3 ml, 10.1 mmol) was added dropwise at -78°C, then the temperature of the mixture was increased to -45°C and stirred for 30 minutes. A THF (10 ml) solution of known 2,3-O-isopropylidene-L-erythrose (Sabino, A. A.; Pilli, R. A., Tetrahedron Lett. 2002, 43, 2819; 808 mg, 5.0 mmol) was added dropwise, then the temperature of the mixture was gradually increased to room temperature over 3.5 hours and stirred overnight. Semisaturated sodium bicarbonate water was added, the mixture was extracted with ethyl acetate, the organic layer was combined, and the resultant mixture was dried over sodium sulfate, then concentrated. The residue obtained was purified by silica gel column chromatography (hexane:ethyl acetate=7:1→5:1) to obtain an intermediate having double bonds in an amount of 707 mg (yield 35%) as a mixture of stereoisomers (E:Z=about 1:5). The obtained intermediate (690 mg, 1.74 mmol) was stirred in ethyl acetate (6 ml) under a hydrogen atmosphere together with a 20% palladium hydroxide (70 mg) for 1 hour. The insolubles were filtered out by a membrane filter and washed well with a chloroform/methanol mixed solution. The filtrate was combined and the resultant mixture was concentrated and purified by silica gel column chromatography (hexane:ethyl acetate=5:1) to obtain the above-identified compound in an amount of 585 mg (yield 84%).

### Example 23: Synthesis of 4R,5R-2,3-O-isopropylidene-2,3-dihydroxydocosanol (Compound 23)

To a dichloromethane (2 ml) solution of oxalyl chloride (92 µl) cooled to -78°C, a dichloromethane (1 ml) solution of dimethyl sulfoxide in an amount of 100 µl was added. The mixture was stirred for 15 minutes, then a dichloromethane (2 ml) solution of the compound 22 synthesized in Example 22 was added and the mixture stirred for 20 minutes. The temperature of the mixture was increased to -45°C, stirred for 1 hour, then triethylamine (600 µl) was added. The temperature of the mixture was then gradually increased to 0°C over 1 hour or more. The mixture was stirred for 20 minutes, then saturated ammonium chloride solution was added at 0°C, the temperature of the mixture was further increased to room temperature, then the mixture was extracted with dichloromethane. The organic layer was combined and the resultant mixture was dried over sodium sulfate and concentrated to obtain the above-identified compound in an amount of 103 mg (yield 57%). The crude product obtained was used for the next process without purification.

### Example 24: Synthesis of 3R,4S,5R-4,5-O-isopropylidene-1-α-D-(tetra-O-benzylgalactopyranosyl)-1-tetracosine-3,4,5-triol (Compound 24)

To a THF (2 ml) solution of known 1-α-ethynyl-2,3,4,6-tetra-O-benzyl-D-galactose (Dondoni, A.; Mariotti, G.; Marra, A. J. Org. Chem. 2002, 67, 4475; 103 mg, 0.188 mmol), a 1.6M n-butyllithium hexane solution (140 µl, 0.224 mmol) was added dropwise at -45°C, then the mixture was stirred for 30 minutes. To this mixture, a THF (3.5 ml) solution of Compound 23 synthesized in Example 23 was added, then the mixture was stirred for 4 hours. A 0.1M phosphate buffer (3 ml) was added at -30°C, then the mixture was gradually raised to room temperature. Further, saline was added, then the resultant mixture was extracted with ethyl acetate, the organic layer was combined, and the resultant mixture was dried over sodium sulfate. The resultant product was concentrated and the residue obtained was purified by silica gel column chromatography (hexane:ethyl acetate=6:1→3:1) to obtain the above-identified compound in an amount of 68.4 mg (yield 38%).

### Example 25: Synthesis of 3R,4S,5R-4,5-O-isopropylidene-1-α-D-(tetra-O-benzylgalactopyranosyl)-tetracosane-3,4,5-triol (Compound 25)

To a dimethoxyethane (3 ml) solution of Compound 24 synthesized in Example 24 (68.4 mg, 0.072 mmol), toluenesulfonyl hydrazide (137.9 mg, 0.74 mmol) was added and the resultant mixture was heated to 85°C. While heating and stirring, a 1N aqueous sodium acetate solution (73 µl, 0.073 mmol) was added every 30 minutes 11 times. After finishing of the addition, the mixture was further stirred for 3 hours. The mixture was cooled to room temperature, then water was added and the resultant mixture was extracted with dichloromethane, the organic layer was combined, and the mixture was dried over sodium sulfate. The resultant product was concentrated and the residue obtained was purified by silica gel column chromatography (hexane:ethyl acetate=4:1) to obtain the above-identified compound in an amount of 55.8 mg (yield 81%).

### Example 26: Synthesis of 3R,4S,5R-4,5-O-isopropylidene-3-methane sulfonyloxy-1-α-D-(tetra-O-benzylgalactopyranosyl)-tetracosane-4,5-diol (Compound 26)

To a dichloromethane/pyridine=2/1 solution of Compound 25 synthesized in Example 25 (55.8 mg, 0.059 mmol), methanesulfonyl chloride (4 drops, about 66 mg) was added at 0°C. The temperature of mixture was gradually increased to room temperature overnight. Methanesulfonyl chloride (10 drops) was further added at room temperature, then after 2.5 hours, methanesulfonyl chloride (10 drops) was further added. The mixture was stirred for 1 hour, then was diluted with ethyl acetate, washed with an aqueous saturated ammonium chloride solution and water, then dried over sodium sulfate. The resultant product was concentrated and the residue obtained was purified by silica gel column chromatography (hexane:ethyl acetate=3:1) to obtain the above-identified compound in an amount of 60.5 mg (yield 100%).

### Example 27: Synthesis of 3S,4S,5R-4,5-O-isopropylidene-3-hexanoylamino-1-α-D-(tetra-O-benzylgalactopyranosyl)-tetracosane-4,5-diol (Compound 27)

To a DMF solution of Compound 26 synthesized in Example 26 (60.5 mg, 0.021 mmol), sodium azide (43.3 mg, 0.67 mmol) was added and the mixture stirred at 90°C overnight. The mixture was diluted with ethyl acetate, washed with water, then dried over sodium sulfate. The resultand mixture was concentrated and the residue obtained was purified by silica gel column chromatography (hexane:ethyl acetate=6:1) to obtain an azide compound. Next, the resultant compound was dissolved in 2-propanol (3 ml) and stirred under a hydrogen atmosphere together with Lindlar's catalyst for 1 day. The reaction was slow and the rate of progress was about 50%, so the catalyst was removed by filtration using a membrane filter, then the filtrate was concentrated, then the entire amount was dissolved in ethanol (3 ml), then the resultant solution was stirred under a hydrogen atmosphere together with Lindlar's catalyst for 1 day. The material substantially entirely was consumed, so the catalyst was removed by filtration using a membrane filter and the filtrate was concentrated to obtain an amine compound. The amine obtained was dissolved in DMF/dichloromethane=1/lsolvent, cerotic acid (7.8 mg, 0.020 mmol), 1-hydroxy-7-azabenzotriazole (4.7 mg, 0.035 mmol), triethylamine (3 drops) and N-dimethylaminopropyl N'-ethyl carbonyl diimidazole hydrochloride (7.1 mg, 0.037 mmol) were added in this order and the resultant mixture was stirred for 3 days at room temperature. Ethyl acetate in an amount of 60 ml was added to dilute this mixture, then the resultant product was washed by saturated sodium bicarbonate water and water and dried over sodium sulfate. The resultant product was concentrated and the residue obtained was purified by silica gel column chromatography (hexane:ethyl acetate=6:1→4:1) to obtain the above-identified compound in an amount of 11.2 mg (yield 40%). "

### Example 28: Synthesis of 3S,4S,5R-1-α-D-galactopyranosyl-3-hexanoylaminotetracosane-9,5-diol (Compound 28)

To a dichloromethane/methanol=5/1 solution (4.8 ml) of Compound 27 synthesized in Example 27 (11.2 mg, 0.0084 mmol), a 4N hydrochloric acid/dioxane solution (200 µl) was added and the mixture stirred for 1.5 hours. This was then cooled to 0°C and neutralized with calcium hydroxide (61.8 mg), then the insolubles were filtered out by a membrane filter. The resultant product was concentrated and the residue obtained was purified by silica gel column chromatography (hexane:ethyl acetate=2:1→1:1) to obtain a body free of isopropylidene in an amount of 9.4 mg. This was dissolved in a dichloromethane/methanol=1/2 solution (4.5 ml) and stirred under a hydrogen atmosphere together with 20% palladium hydroxide (11 mg) for 5.5 hours. The insolubles were filtered out by a membrane filter and washed well with a chloroform/methanol mixed solution. The filtrate was combined and the resultant comopund was condensed to obtain the above-identified compound in an amount of 7.7 mg (yield 98%).

The physicochemical data obtained by the above Examples are shown in Table I.

**Table I**

| Compound no. | Chemical formula | Rf value | MS (FAB) | ¹H-NMR | |
|---|---|---|---|---|---|
| 1 | 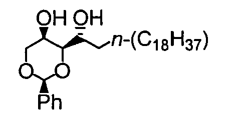 | Hex:EA=1: 1 Rf=0.5 | 477 (M) | <CDC13>7.55-7.45 (m, 2H), 7.45-7.30 (m, 3H), 5.59 (s, 1H), 4.27 (dd, 1H, J=12.4Hz, 1.9Hz), 4.05 (dd, 1H, J=12.0Hz, 1.3Hz), 4.0-3.9 (m, 1H), 3.89 (dd, 1H, J=8.7Hz, 1.5Hz), 3.70 (dd, 1H, J=6.7Hz, 1.3Hz), 3.24 (d, 1H, 8.7Hz), 2.31 (d, 1H, J=4.5Hz), 1.8-1.65 (m, 1H), 1.65-1.5 (m, 1H), 1.5-1.2 (m, 34H), 0.88 (t, 3H, J=6.6Hz) | |
| 2 | 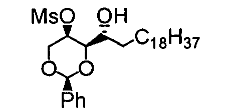 | Hex:EA=1: 1 Rf=0.7 | 555 (M) | <CDCl3>7.55-7.45 (m, 2H), 7.45-7.30 (m, 3H), 5.59 (s, 1H), 4.98 (d, 1H, 1.4Hz), 4.52 (dd, 1H, J=13.2Hz, 1.6Hz), 4.16 (dd, 1H, J=13.2Hz, 1.3Hz), 3.9-3.75 (m, 1H), 3.75 (dd, 1H, J=8.9Hz, 1.3Hz), 3.19 (s, 3H), 2.77 (d, 1H, 5.0Hz), 1.95-1.8 (m, 1H), 1.65-1.3 (m, 35H), 0.88 (t, 3H, J=6.7Hz) | |
| 3 | 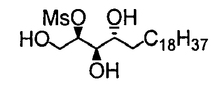 | Hex:EA=1: 1 Rf=0.3 tailing | 467 (M) | <DMSO-d6> 4.73 (ddd, 1H, J=6.4Hz, 6.4Hz, 1.4Hz), 3.7-3.55 (m, 2H), 3.45-3.2 (m, 2H), 3.16 (s, 3H), 1.75-1.6 (m, 1H), 1.55-1.35 (m, 1H), 1.35-1.2 (m, 34H), 0.85 (t, 3H, J=6.6Hz) | |
| 4 | 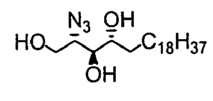 | Hex:EA=1: 2 Rf=0.5 | 414 (M) | <DMSO-d6> 4.98 (d, 1H, J=5.8Hz), 4.86 (dd, 1H, J=5.6Hz, 4.7Hz), 4.50 (d, 1H, J=6.5Hz), 3.75 (ddd, 1H, J=11.1Hz, 4.5Hz, 3.0Hz) 3.65-3.55 (m, 1H), 3.65-3.55 (ddd, 1H, J=11.0Hz, 5.8Hz), 3.52 (ddd, 1H, J=9.0Hz, 3.5Hz, 3.0Hz) 3.4-3.3 (m, 1H), 1.6-1.35 (m, 2H), 1.35-1.2 (m, 34H), 0.85 (t, 3H, J=6.6Hz) | |
| 5 | 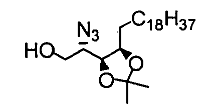 | Hex:EA=6 : 1 Rf=0.25 | 426 (M-N2) | <CDCl3>4.18 (ddd, 1H, J=9.5Hz, 5.6Hz, 3.6Hz), 3.99 (ddd, 1H, J=11.6Hz, 5.4Hz, 4.3Hz) 3.97 (dd, 1H, J=9.3Hz, 5.7Hz), 3.87 (ddd, 1H, J=11.6Hz, 6.9Hz, 5.6Hz), 3.47 (ddd, 1H, J=9.3Hz, 5.4Hz, 4.4Hz), 2.07 (dd, 1H, J=6.9Hz, 5.6Hz), 1.7-1.5 (m, 2H), 1.43(s, 3H), 1.34(s, 3H), 1.5-1.2 (m, 34H), 0.88 (t, 3H, J=6.7Hz) | |
| 6 | 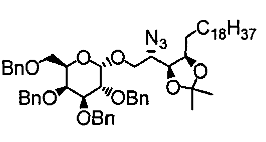 | Hex:EA=3 : 1 Rf=0.6 | 975 (M - 1) 998 (M + Na - 1) | <CDC13>7.4-7.2 (m, 20H), 4.94 (d, 1H, J=11.4Hz), 4.94 (d, 1H, J=3.5Hz), 4.84 (d, 1H, J=11.8Hz), 4.79 (d, 1H, J=12.0Hz), 4.71 (d, 1H, J=11.8Hz), 4.70 (d, 1H, J=12.0Hz), 4.56 (d, 1H, J=11.5Hz), 4.48 (d, 1H, J=12.0Hz), 4.40 (d, 1H, J=12.0Hz), 4.15-3.95 (m, 6H), 3.95-3.9 (m, 1H), 3.71 (dd, 1H, J=10.8Hz, 6.7Hz), 3.50 (dd, 1H, J=6.1Hz, 3.0Hz), 3.55-3.45 (m, 2H), 1.7-1.45 (m, 2H), 1.37 (s, 3H), 1.27 (s, 3H), 1.45-1.2 (m, 34H), 0.88 (t, 3H, J=6.6Hz) | |
| 7 | 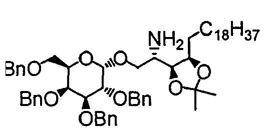 | Hex:EA=3 : 1 Rf=0.1 tailing | 951 (M + 1) | <CDC13>7.4-7.2 (m, 20H), 4.92 (d, 1H, J=11.4Hz), 4.90 (d, 1H, J=3.7Hz), 4.82 (d, 1H, J=11.6Hz), 4.80 (d, 1H, J=11.7Hz), 4.75 (d, 1H, J=11.7Hz), 4.67 (d, 1H, J=11.6Hz), 4.55 (d, 1H, J=11.5Hz), 4.49 (d, 1H, J=11.8Hz), 4.40 (d, 1H, J=11.8Hz), 4.2-4.1 (m, 2H), 4.06 (dd, 1H, J=9.9Hz, 3.7Hz), 4.05-3.9 (m, 4H), 3.52 (d, 2H, J=6.4Hz), 3.46 (dd, 1H, J=10.6Hz, 8.0Hz), 3.25-3.15 (m, 1H), 1.6-1.45 (m, 2H), 1.38 (s, 3H), 1.28 (s, 3H), 1.45-1.2 (m, 34H), 0.88 (t, 3H, J=6.7Hz) | |
| 8 | 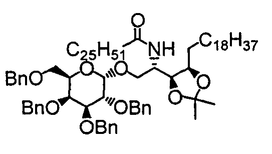 | Hex:EA=3 : 1 Rf=0.6 | 1328 (M - 1) | <CDCl3>7.4-7.2 (m, 20H), 6.28 (d, 1H, J=8.6Hz), 4.93 (d, 1H, J=11.6Hz), 4.90 (d, 1H, J=3.8Hz), 4.81 (d, 1H, J=11.7Hz), 4.80 (d, 1H, J=11.5Hz), 4.74 (d, 1H, J=11.7Hz), 4.63 (d, 1H, J=11.5Hz), 4.58 (d, 1H, J=11.6Hz), 4.49 (d, 1H, J=11.8Hz), 4.37 (d, 1H, J=11.8Hz), 4.2-4.02 (m, 4H), 4.02-3.95 (m, 1H), 3.95-3.9 (m, 3H), 3.65-3.58 (m, 1H), 3.55 (dd, 1H, J=9.3Hz, 7.0Hz), 3.38 (dd, 1H, J=9.4Hz, 5.6Hz), 2.15-1.95 (m, 2H), 1.7-1.2 (m, 82H), 1.40 (s, 3H), 1.31(s, 3H), 0.88 (t, 6H, J=6.6Hz) | |
| 9 | 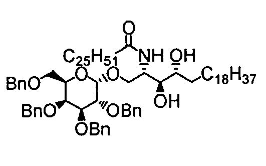 | Hex:EA=2 : 1 Rf=0.3 | 1289 (M) | <CDC13>7.4-7.2 (m, 20H), 6.38 (d, 1H, J=8.3Hz), 4.91 (d, 1H, J=11.5Hz), 4.87 (d, 1H, J=11.6Hz), 4.84 (d, 1H, J=3.8Hz), 4.77 (d, 1H, J=11.8Hz), 4.74 (d, 1H, J=11.8Hz), 4.67 (d, 1H, J=11.6Hz), 4.56 (d, 1H, J=11.5Hz), 4.47 (d, 1H, J=11.6Hz), 4.38 (d, 1H, J=11.6Hz), 4.25-4.15 (m, 1H), 4.04 (dd, 1H, J=10.0Hz, 3.7Hz), 4.0-3.95 (m, 1H), 3.95-3.84 (m, 4H), 3.84-3.75 (m, 1H), 3.56-3.44 (m, 4H), 2.11 (dd, 1H, J=8.2Hz, 7.0Hz), 1.6-1.2 (m, 82H), 0.88 (t, 6H, J=6.6Hz) | |
| 10 | 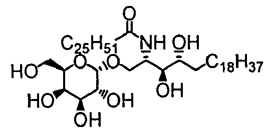 | CH2Cl2:MeOH=10 : 1 Rf=0.1 | 950 (M + Na -1) | <Pyridine-d5>8.47 (d, 1H, J=8.7Hz), 5.59 (d, 1H, J=3.8Hz), 5.32-5.2 (m, 1H), 4.69 (dd, 1H, J=11.0Hz, 5.3Hz), 4.66 (dd, 1H, J=9.7Hz, 3.9Hz), 4.56 (dd, 1H, J=3.2Hz), 4.53 (dd, 1H, J=6.1Hz), 4.5-4.35 (m, 4H), 4.35-4.3 (m, 2H), 2.45 (t, 2H, J=7.4Hz), 2.4-2.2 (m, 1H), 2.0-1.75 (m, 4H), 1.75-1.6 (m, 1H), 1.6-1.2 (m, 76H), 0.88 (t, 6H, J=6.7Hz) | |
| 11 | 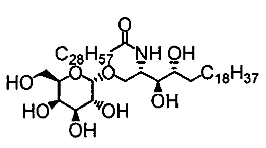 | CH2Cl2:MeOH=10 : 1 Rf=0.1 | FAB 971 (M) | <Pyridine-d5>8.47 (d, 1H, J=8.9Hz), 7.0-6.9 (m, 1H), 6.7-6.58 (m, 1H), 6.58-6.5 (m, 1H), 6.5-6.4 (m, 1H), 6.35-6.25 (m, 1H), 6.15-6.05 (m, 1H), 5.59 (d, 1H, J=3.8Hz), 5.32-5.2 (m, 1H), 4.69 (dd, 1H, J=11.0Hz, 5.3Hz), 4.73-4.63 (m, 1H), 4.6-4.57 (m, 1H), 4.53 (dd, 1H, J=6.1Hz, 6.0Hz), 4.5-4.35 (m, 4H), 4.35-4.3 (m, 2H), 2.45 (t, 2H, J=7.1Hz), 2.4-2.2 (m, 1H), 2.0-1.75 (m, 4H), 1.75-1.6 (m, 1H), 1.6-1.2 (m, 82H), 0.88 (t, 6H, J=6.7Hz) | |

| Compound no. | Chemical formula | Rf value | MS (MALDI) | Elementary analysis | ¹H-NMR |
|---|---|---|---|---|---|
| 12 | 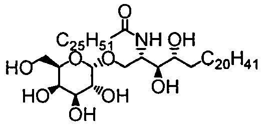 | CHC13:MeOH=7 : 1 Rf=0.19 | 979.23 (M + Na) | Obsd C, 69.21; H, 11.50; N, 1.40 Calcd for C57H113NO9_1.8H2O C, 69.23; H, 11.88; N, 1.42 | <CDCl3:CD3OD=3:1> 4.90 (d, 1H), 4.3-4.15 (m, 1H), 3.95-3.5 (m, 11H), 2.22 (t, 2H), 1.8-1.2 (m, 86H), 0.88 (t, 6H) |
| 13 | 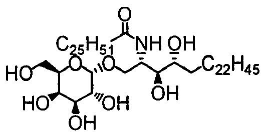 | CHCl3:MeOH=7 : 1 Rf=0.24 | 1007.41 (M + Na) | Obsd C, 69.37; H, 11.88; N, 1.28 Calcd for C59H117NO9_2H2O C, 69.43; H, 11.95; N, 1.37 | <CDC13:CD30D=3:1> 4.90 (d, 1H), 4.3-4.15 (m, 1H), 3.95-3.5 (m, 11H), 2.22 (t, 2H), 1.8-1.2 (m, 90H), 0.88 (t, 6H) |
| 14 | 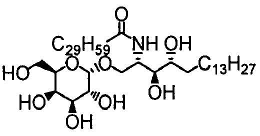 | CHCl3:MeOH=7 : 1 Rf=0.35 | 937.39 (M + Na) | Obsd C, 68.01; H, 11.76; N, 1.54 Calcd for C54H107NO9_2H2O_0.1(C2H6SO) C, 67.93; H, 11.74; N, 1.46 | <CDC13:CD30D=3:1> 4.90 (d, 1H), 4.3-4.15 (m, 1H), 3.95-3.5 (m, 11H), 2.22 (t, 2H), 1.8-1.2 (m, 80H), 0.88 (t, 6H) |
| 15 | 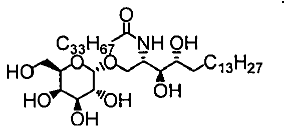 | CHCl3:MeOH=7 : 1 Rf=0.23 | 993.14 (M + Na) | Obsd C, 69.90; H, 11.86; N, 1.30 Calcd for C58H115NO9_1.4H2O C, 69.96; H, 11.92; N, 1.41 | <CDCl3:CD30D=3:1> 4.90 (d, 1H), 4.3-4.15 (m, 1H), 3.95-3.5 (m, 11H), 2.22 (t, 2H), 1.8-1.2 (m, 88H), 0.88 (t, 6H) |

| Compound no. | Chemical formula | Rf value | MS (FAB) | ¹H-NMR | |
|---|---|---|---|---|---|
| 16 | 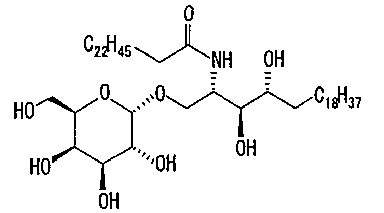 | CH₂Cl₂:MeOH =10:1 Rf=0.1 | 900 (M) | 8.48 (d, 1H, J=8.7Hz), 5.56 (d, 1H, J=3.7Hz), 5.31-5.22 (m, 1H), 4.67 (dd, 1H, J=11.0Hz, 5.2Hz), 4.65 (dd, 1H, J=9.7Hz, 3:6Hz), 4.58-4.56 (m, 1H), 4.52 (dd, 1H, J=6.1Hz), 4.5-4.35 (m, 4H), 4.35-4.3(m, 2H), 2.43 (t, 2H, J=7.3Hz), 2.4-2.2 (m, 1H), 2.0-1.75 (m, 4H), 1.75-1.6 (m, 1H), 1.6-1.2 (m, 72H), 0.86 (t, 6H, J=6.7Hz) | |
| 17 | 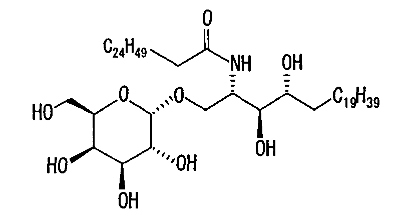 | CH₂Cl₂:MeOH =10:1 Rf=0.2 | 965 (M+Na) | 8.47 (d, 1H, J=8.8Hz), 7.0-6.8 (m, 1H), 6.7-6.0 (m, 5H), 5.59 (d, 1H, J=3.8Hz), 5.32-5.22 (m, 1H),4.69 (dd, 1H, J=11.0Hz, 5.2Hz), 4.73-4.63 (m, 1H),4.6-4.56 (m, 1H), 4.53 (dd, 1H, J=6.1Hz, 6.0Hz), 4.5-4.38 (m, 4H), 4.38-4.3 (m, 2H), 2.45 (t, 2H, J=7.3Hz), 2.4-2.2 (m, 1H), 2.0-1.78 (m, 4H), 1.78-1.6 (m, 1H), 1.6-1.2 (m, 78H), 0.88 (t, 6H, J=6.7 Hz) | |
| 18 | 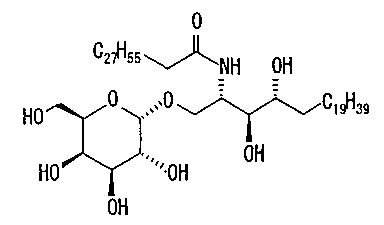 | CH₂Cl₂:MeOH =10:1 Rf=0.2 | 985 (M) | 8.47 (d, 1H, J=8.7Hz), 7.05-6.9 (m, 1H), 6.7-6.5 (m, 2H), 6.5-6.4 (m, 1H), 6.35-6.25 (m, 1H), 6.1-6.0 (m, 1H), 5.59 (d, 1H, J=3.9Hz), 5.32-5.22 (m, 1H), 4.69 (dd, 1H, J=10.9Hz, 5.1Hz), 4.73-4.63 (m,1H), 4.6-4.57 (m, 1H), 4.53 (dd, 1H, J=6.7Hz, 5.7 Hz), 4.5-4.38 (m, 4H), 4.38-4.3 (m, 2H), 2.45 (t, 2H, J=7.2Hz), 2.4-2.2 (m, 1H), 2.05-1.75 (m, 4H), 1.75-1.6 (m, 1H), 1.6-1.2 (m, 84H), 0.88 (t, 6H, J=6.7Hz) | |
| 19 | 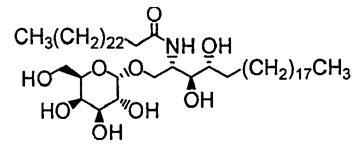 | CH2C12:MeOH=5 : 1 Rf=0.6 | 913 (M-1) | <Pyridine-d5>8.50 (d, 1H, J=9.1Hz), 5.59 (d, 1H, J=3.8Hz), 5.33-5.22 (m, 1H), 4.73-4.63 (m, 2H), 4.6-4.56 (m, 1H), 4.53 (dd, 1H, J=6.0Hz, 6.0Hz), 4.5-4.38 (m, 4H), 4.38-4.3 (m, 2H), 2.46 (t, 2H, J=7.2Hz), 2.4-2.2 (m, 1H), 2.05-1.8 (m, 4H), 1.8-1.6 (m, 1H), 1.6-1.2 (m, 74H), 0.88 (t, 6H, J=6.6Hz) | |
| 20 | 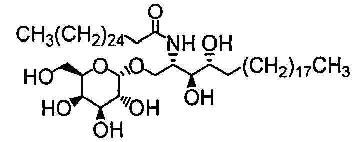 | CH2Cl2:MeOH=5 : 1 Rf=0.6 | 942 (M) | <Pyridine-d5>8.50 (d, 1H, J=9.0Hz), 7.1-6.0 (m, 6H), 5.59 (d, 1H, J=3.8Hz), 5.33-5.22 (m, 1H), 4.75-4.63 (m, 2H), 4.6-4.56 (m, 1H), 4.53 (dd, 1H, J=6.4Hz, 6.4Hz), 4.5-4.38 (m, 4H), 4.38-4.3 (m, 2H), 2.46 (t, 2H, J=7.3Hz), 2.4-2.2 (m, 1H), 2.05-1.8 (m, 4H), 1.8-1.6 (m, 1H), 1.6-1.2 (m, 78H), 0.88 (t, 6H, J=6.5Hz) | |
| 21 | 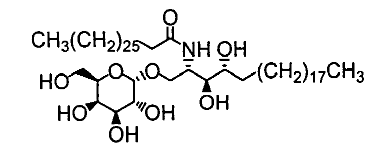 | CH2Cl2:MeOH=5 : 1 Rf=0.6 | 956 (M-1) | <Pyridine-d5>8.52 (d, 1H, J=9.2Hz), 5.59 (d, 1H, J=3.9Hz), 5.33-5.22 (m, 1H), 4.75-4.65 (m, 2H), 4.6-4.56 (m, 1H), 4.53 (dd, 1H, J=5.6Hz, 5.6Hz), 4.5-4.38 (m, 4H), 4.38-4.3 (m, 2H), 2.46 (t, 2H, J=7.2Hz), 2.4-2.2 (m, 1H), 2.05-1.8 (m, 4H), 1.8-1.6 (m, 1H), 1.6-1.2 (m, 80H), 0.88 (t, 6H, J=6.7Hz) | |
| 22 | 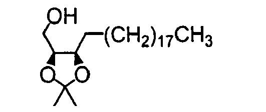 | Hexane:AcOEt=3:1 Rf=0.5 | 399 (M) | <CDCl3>4.2-4.1(m, 2H), 3.7-3.55(m, 2H), 1.84(dd, 1H, J=7.1Hz, 5.0Hz), 1.47(s, 3H), 1.37(s, 3H), 1.35-1.2(m, 36H), 0.88(t, 3H, 6.7Hz) | |
| 23 | 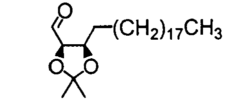 | Hexane:AcOEt=3:1 Rf=0.8 | 395 (M-2) | <CDCl3>9.63(d, 1H, J=3.6Hz), 4.4-4.3(m, 1H), 4.24(dd, 1H, J=7.1Hz, 3.5Hz), 1.59(s, 3H), 1.42(s, 3H), 1.55-1.2(m, 36H), 0.88(t, 3H, 6.6Hz) | |
| 24 | 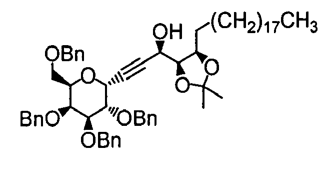 | Hexane:AcOEt=3:1 Rf=0.3 | 945 (M) | <CDCl3>7.4-7.2(m, 20H), 4.91(d, 1H, J=11.4Hz), 4.87(dd, 1H, J=5.8Hz, 1.6Hz), 4.81(d, 1H, J=11.7Hz), 4.74(d, 1H, J=11.7Hz), 4.72(d, 1H, J=11.7Hz), 4.67(d, 1H, J=11.8Hz), 4.55(d, 1H, J=11.4Hz), 4.48(d, 1H, J=11.8Hz), 4.40(d, 1H, J=11.8Hz), 4.36(dd, 1H, J=7.2Hz, 1.6Hz), 4.2-4.0(m, 4H), 4.0-3.9(m, 1H), 3.82(dd, 1H, J=9.8Hz, 2.8Hz), 3.52(d, 2H, J=6.2Hz), 2.8-2.5(m, 2H), 1.47(s, 3H), 1.37(s, 3H), 1.35-1.2(m, 36H), 0.88(t, 3H, 6.7Hz) | |
| 25 | 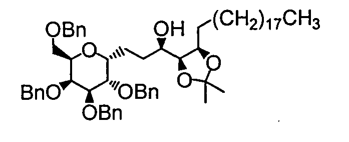 | Hexane:AcOEt=3:1 Rf=0.4 | 949 (M) | <CDC13>7.4-7.2(m, 20H), 4.76(d, 1H, J=11.7Hz), 4.69(d, 1H, J=12.0Hz), 4.64(d, 2H, J=13.2Hz), 4.56(d, 1H, J=11.7Hz), 4.55(d, 1H, J=11.8Hz), 4.50(d, 1H, J=12.0Hz), 4.44(d, 1H, J=11.9Hz), 4.1-3.9(m, 4H), 3.9-3.8(m, 1H), 3.83(dd, 1H, J=6.0Hz, 4.7Hz), 3.8-3.7(m, 1H), 3.73(dd, 1H, J=7.6Hz, 2.5Hz), 3.7-3.55(m, 2H), 2.3-2.1(bs, 1H), 2.0-1.8(m, 1H), 1.8-1.6(m, 2H), 1.46(s, 3H), 1.34(s, 3H), 1.6-1.2(m, 37H), 0.88(t, 3H, 6.6Hz) | |
| 26 | 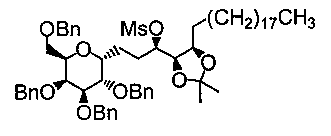 | Hexane:AcOEt=3:1 Rf=0.5 | 1027 (M) | <CDCl3>7.4-7.2(m, 20H), 4.8-4.7(m, 2H), 4.68(s, 2H), 4.60(s, 2H), 4.55(d, 1H, J=11.7Hz), 4.49(d, 1H, J=11.9Hz), 4.43(d, 1H, J=12.0Hz), 4.08(dd, 1H, J=8.3Hz, 5.5Hz), 4.15-4.05(m, 1H), 4.0-3.9(m, 2H), 3.9-3.8(m, 2H), 3.8-3.65(m, 1H), 3.72(dd, 1H, J=7.7Hz, 2.7Hz), 3.56(dd, 1H, J=10.3Hz, 4.8Hz), 3.08(s, 3H), 2.0-1.7(m, 3H), 1.7-1.5(m, 2H), 1.44(s, 3H), 1.33(s, 3H), 1.4-1.2(m, 35H), 0.88(t, 3H, 6.7Hz) | |
| 27 | 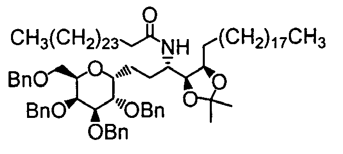 | Hexane:AcOEt=3:1 Rf=0.6 | 1329 (M+2) | <CDC13>7.4-7.2(m, 20H), 5.69(d, 1H, J=8.7Hz), 4.74(d, 1H, J=11.7Hz), 4.68(d, 1H, J=11.8Hz), 4.64(d, 1H, J=11.9Hz), 4.63(d, 1H, J=11.9Hz), 4.54(d, 1H, J=10.3Hz), 4.52(d, 1H, J=11.9Hz), 4.45(d, 1H, J=12.0Hz), 4.1-3.9(m, 6H), 3.9-3.73(m, 2H), 3.68(dd, 1H, J=7.7Hz, 2.6Hz), 3.54(dd, 1H, J=10.5Hz, 4.6Hz), 2.15-2.0(m, 2H), 1.40(s, 3H), 1.30(s, 3H), 1.9-1.2(m, 89H), 0.88(t, 6H, 6.7Hz) | |
| 28 | 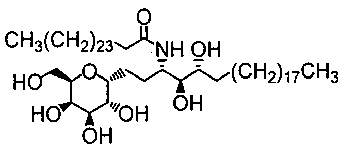 | CH2Cl2:MeOH=10:1 Rf=0.2 | 926 (M) | <Pyridine-d5>8.47(d, 1H J=9.0Hz), 5.2-5.05(m, 1H), 4.74(dd, 1H, J=9.0Hz, 5.3Hz), 4.6-4.45(m, 3H), 4.37(dd, 1H, J=11.4Hz, 4.5Hz), 4.3-4.2(m, 4H), 2.83-2.7(m, 1H), 2.7-2.55(m, 1H), 2.55-2.4(m, 2H), 2.4-2.1(m, 3H), 2.05-1.8(m, 4H), 1.8-1.55(m, 1H), 1.45-1.1(m, 78H), 0.88(t, 6H, 6.6Hz) | |

### Suppressive Effect of Synthesized Glycolipid

### Derivative in K/Bxn Serum Transferred Arthritis

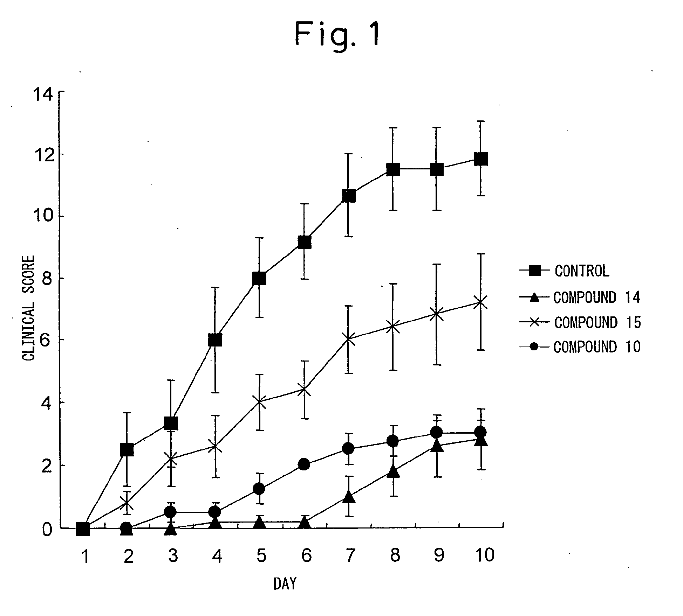

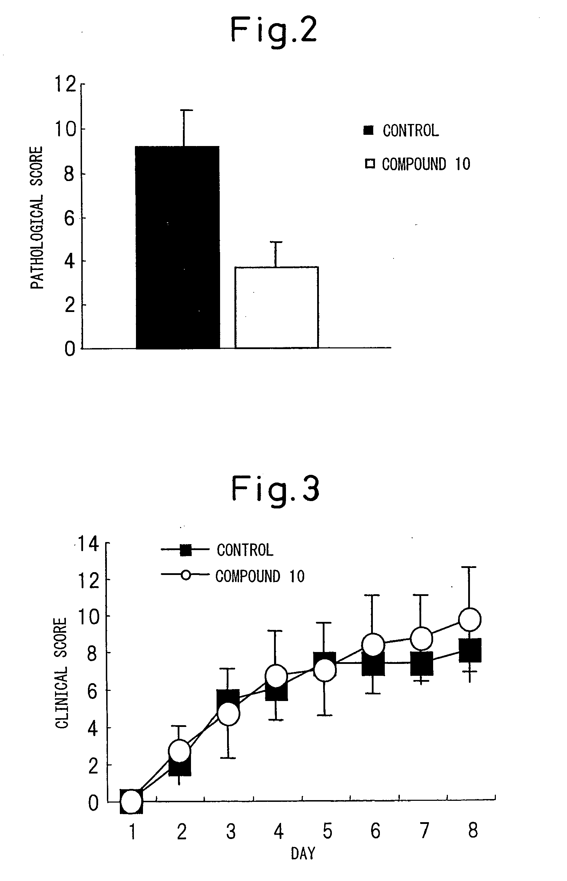

(A) C57BL/6J mice (8 weeks old, female) were intraperitoneally administered K/BxN serum in amounts of 150 µl to induce arthritis. The arthritis score was judged by observation in the following way.
   The arthritis was scored as follows:
   0: no symptoms,
   1: swelling and reddening of only one small joint of digits of limb,
   2: swelling and reddening of two or more small joints or a relatively large joint such as a wrist or ankle,
   3: swelling and reddening of an arm or leg as a whole,
   4: overall swelling of one arm or leg reaching the maximum extent.
   The total of the two arms and two legs was used as the score. The compound was dissolved in 10% DMSO/PBS and intraperitoneally administered as 2 µg/mouse twice a week from the day of administration of the serum.
   For the control group, only 10% DMSO/PBS was administered. As a result of the experiment, the administration of the compound enabled the arthritis score to be remarkably suppressed (see FIG. 1).
(B) The mice used for the experiment were examined for pathological tissue on the eighth day after serum transfer. The pathological score was judged as follows: The pathology was scored as
   0: normal,
   1: light arthritis with synovial inflammation, but with no cartilage and bone lesions,
   2: medium arthritis accompanied with synovial inflammation and cartilage and bone lesions,
   3: severe arthritis with advanced synovial inflammation and cartilage and bone lesions.
      The total of the two arms and two legs was used as the score. As a result of the experiment, the administration of the compound enabled the pathological score to be remarkably suppressed (see FIG. 2).
(C) In Jα18 gene-deficient mice with no NKT cells, no effect of suppression of K/BxN serum transferred arthritis in the compound was observed. Therefrom, it was learned that NKT cells are essential for the expression of the suppressive effect on arthritis of the compound according to the present invention (see FIG. 3).

### Effect of Compound on NKT Cells

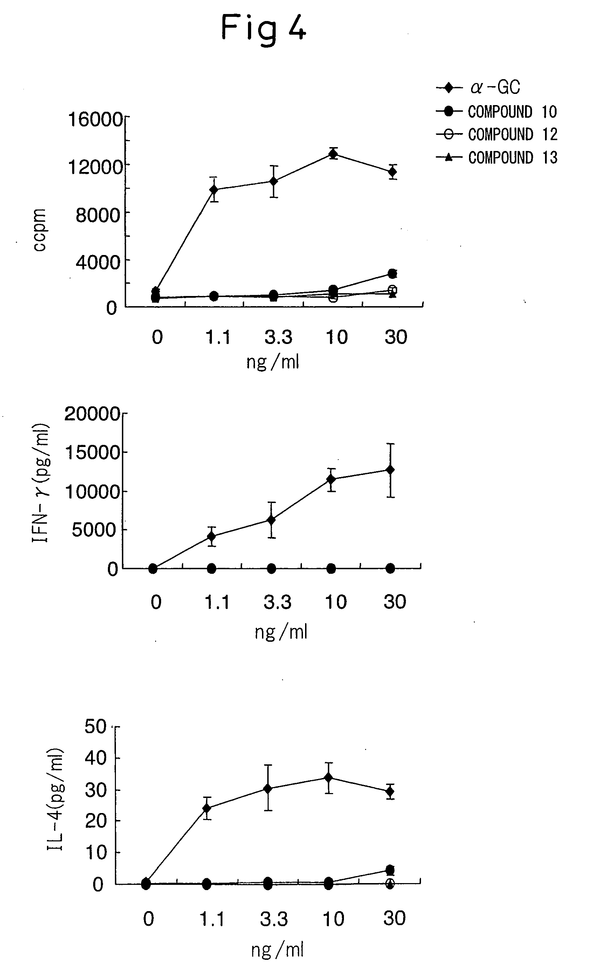

Mononuclear cells were separated from the livers of C57BL/6J mice (8 weeks old, female) and incubated with the compound for 48 hours. The cytokine in the supernatant was measured by the ELISA method, while the cell proliferation reaction was measured by the intake of tritium thymidine. α-GC caused cell proliferation, IFN-γ production and IL-4 production, but the compound did not react in any way. The reaction by α-GC and slight intake of tritium and the slight amount of cytokine production observed for the compound at 30 ng/ml were not seen in Jα18 gene-deficient mice with no NKT cells, and, therefore, these reactions were confirmed to be NKT cell-dependent reactions (see FIG. 4).

### Effect of Preadministration of Compound on NKT Cells

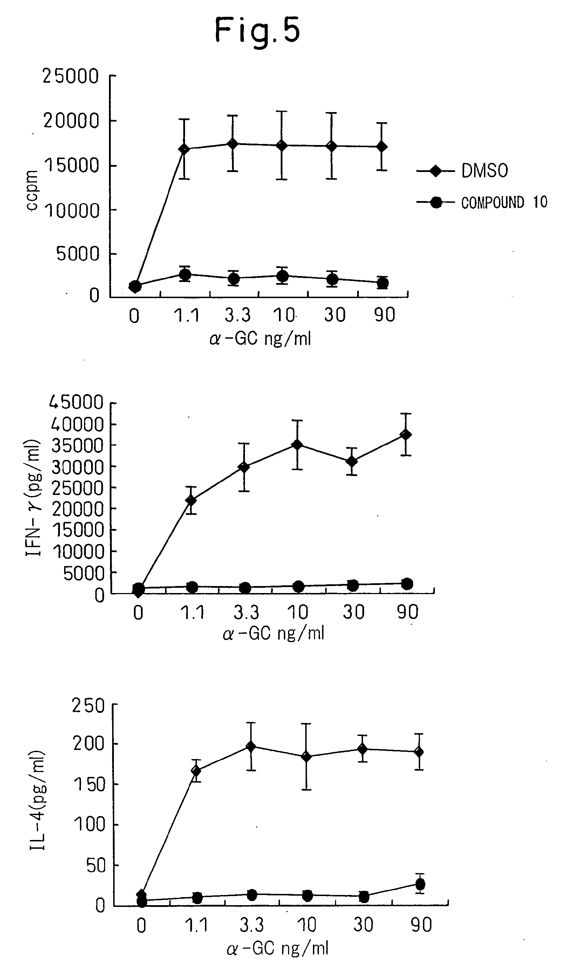

C57BL/6J mice (8 weeks old, female) were intraperitoneally administered with the compound three times every two days in amounts of 2 µg/mouse. For the control group, only 10% DMSO/PBS was administered. Two days after the final administration, mononuclear cells were separated from the livers and incubated together with α-GC for 48 hours, the cytokine in the supernatant was measured by the ELISA method, and the cell proliferation reaction was measured by intake of tritium thymidine. In the group preadministered with the compound, cell proliferation due to α-GC, IFN-γ production, and IL-4 production could not be observed. From this, it became clear that the present invention compound suppressed the reaction with respect to antigen stimulation of NKT cells (see FIG. 5).

### Suppression of Cellular Infiltration in Alveolus Washings in Bronchial Asthma Model

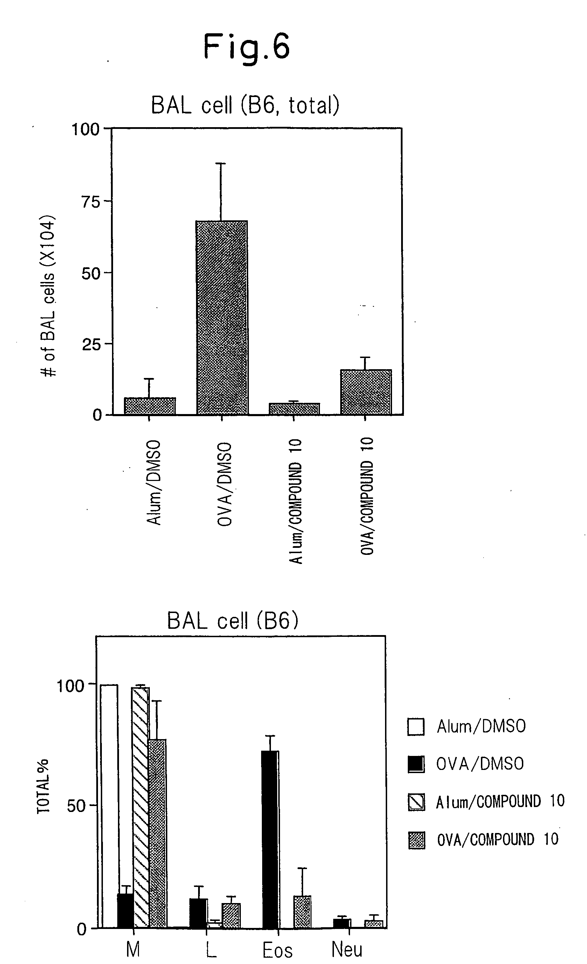

C57BL/6J mice (8 weeks old, female) were intraperitoneally administered with ovalubumin (OVA) in amounts of 50 µg/mouse after blending with alum in an amount of 2.25 mg/mouse on Day 0 and Day 7. Starting from Day 18, the mice were made to inhale OVA for three consecutive days at a concentration of 10 mg/ml. The compound was intraperitoneally administered before inhalation in an amount of 2 µg/mouse. On the day after the final inhalation day, the alveoli were washed and the cell compositions were studied. The cellular infiltration was remarkably suppressed compared with the control group (OVA/DMSO) in the compound group. Further, the acidocyte infiltration characteristic of asthma was remarkably suppressed (see FIG. 6).

### Suppression of Cytokine in Alveolus Washings In Bronchial Asthma Model

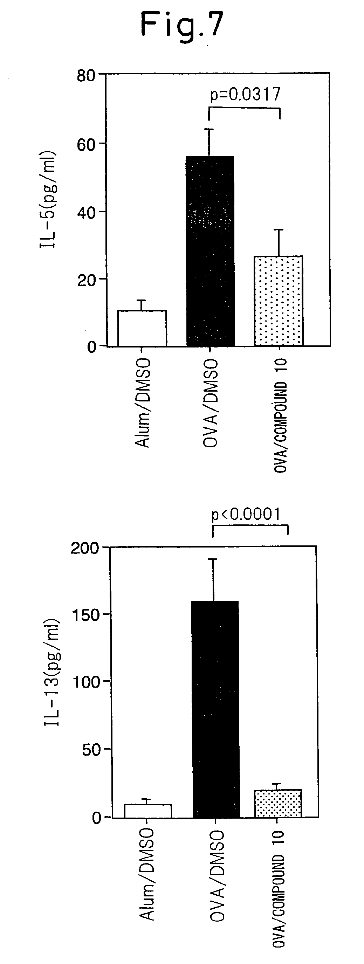

C57BL/6J mice (8 weeks old, female) were intraperitoneally administered with OVA in amounts of 50 µg/mouse after blending with alum in an amount of 2.25 mg/mouse on Day 0 and Day 7. Starting from day 18, the mice were made to inhale OVA for three consecutive days at a concentration of 10 mg/ml. The compound was intraperitoneally administered before inhalation in an amount of 2 µg/mouse. On the day after the final inhalation day, the alveoli were washed and the cytokine in the alveolus washings was measured using the ELISA method. Compared with the control group (OVA/DMSO), IL-5 and IL-13 were both remarkably suppressed in the compound group (see FIG. 7).

### Suppression of Airway Resistance in Bronchial Asthma Model

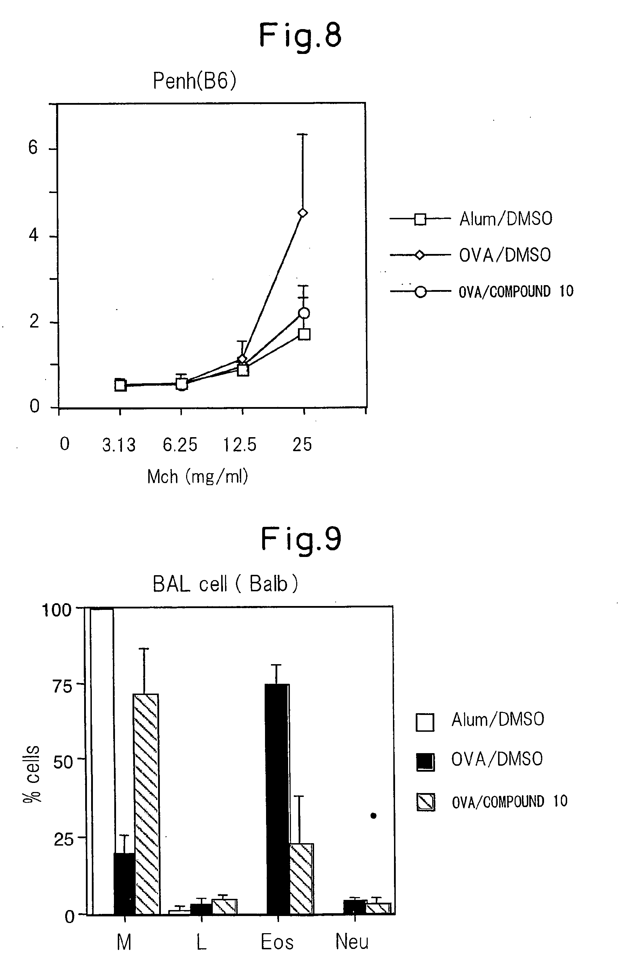

C57BL/6J mice (8 weeks old, female) were intraperitoneally administered with OVA in amounts of 50 µg/mouse after blending with alum in an amount of 2.25 mg/mouse on Day 0 and Day 7. Starting from Day 18, the mice were made to inhale OVA three times every other day at a concentration of 10 mg/ml. The compound was intraperitoneally administered before inhalation in an amount of 2 µg/mouse. On the day after the final inhalation day, the methacholine-induced airway resistance was measured using plethysmography. Compared with the control group (OVA/DMSO), airway resistance was suppressed in the compound group (see FIG. 8).

### Suppression of Cellular Infiltration in Alveolus Washings in Bronchial Asthma Model

Balb/c mice (8 weeks old, female) were intraperitoneally administered with ovalubumin (OVA) in amounts of 20 µg/mouse after blending with alum in an amount of 2.25 mg/mouse on Day 0 and Day 7. Starting from day 18, the mice were made to inhale OVA for three consecutive days at a concentration of 5 mg/ml. The compound was intraperitoneally administered before inhalation in an amount of 2 µg/mouse. On the day after the final inhalation day, the alveoli were washed and the cell compositions were studied. The cellular infiltration was remarkably suppressed compared with the control group (OVA/DMSO) in the compound group. Further, the acidocyte infiltration characteristic of asthma was remarkably suppressed (see FIG. 9).

### Suppression of Cytokine in Bronchial Asthma Model

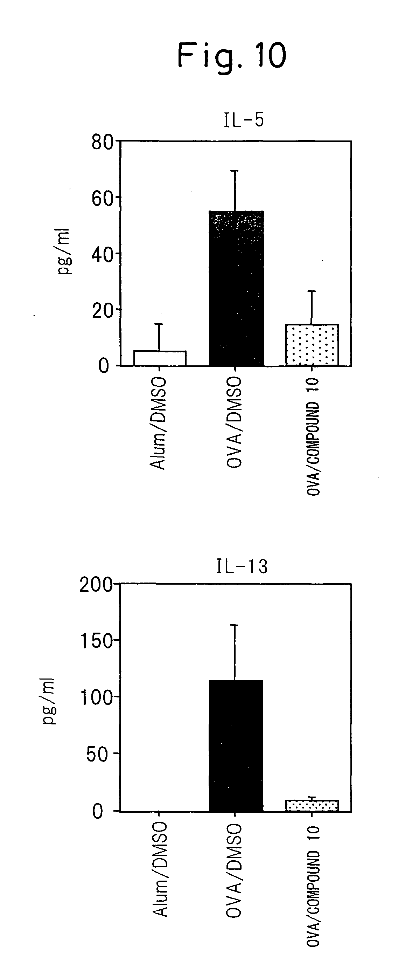

Balb/c mice (8 weeks old, female) were intraperitoneally administered with OVA in amounts of 20 µg/mouse after blending with alum in an amount of 2.25 mg/mouse on Day 0 and Day 7. Starting from day 18, the mice were made to inhale OVA for three consecutive days at a concentration of 5 mg/ml. The compound was intraperitoneally administered before inhalation in an amount of 2 µg/mouse. On the day after the final inhalation day, the alveoli were washed and the cytokine in the alveolus washings was measured using the ELISA method. Compared with the control group (OVA/DMSO), IL-5 and IL-13 were both remarkably suppressed in the compound group (see FIG. 10).

### Suppressive Effects on Airway Resistance in Bronchial Asthma Model

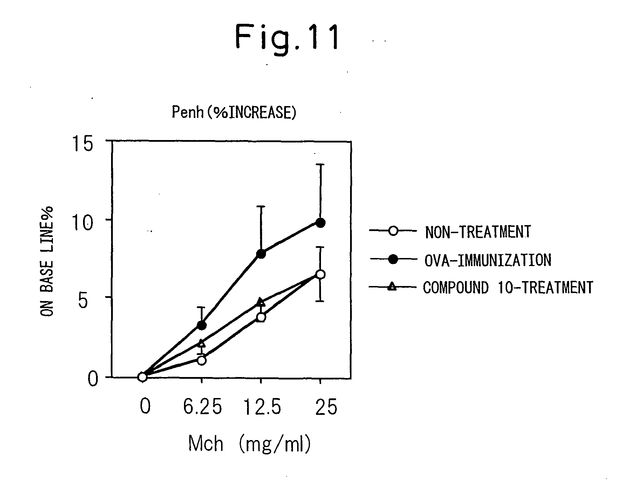

Balb/c mice (8 weeks old, female) were intraperitoneally administered with OVA in amounts of 20 µg/mouse after blending with alum in an amount of 2.25 mg/mouse on Day 0 and Day 7. Starting from Day 18, the mice were made to inhale OVA three times every other day at a concentration of 5 mg/ml. The compound was administered intraperitoneally in amounts of 2 µg/mouse before inhalation. On the day after the final inhalation day, the methacholine-induced airway resistance was measured using plethysmography. Compared with the control group (OVA/DMSO), airway resistance was suppressed in the compound group (see FIG. 11).

### INDUSTRIAL APPLICABILITY

The glycolipid derivative ASG having the formula (I) according to the present invention suppresses autoantibody-induced inflammation reactions and is useful as a drug for treatment of autoimmune arthritis and other autoimmune diseases and bronchial asthma and otherallergic diseases.

## Claims

1. A glycolipid derivative having the formula (I): 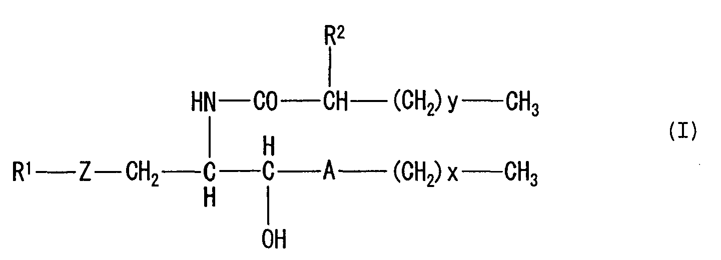 wherein R¹ indicates an aldopyranose residue, R² indicates a hydrogen atom or hydroxyl group, A indicates -CH₂-, -CH(OH)-CH₂- or -CH=CHCH₂-, Z indicates -O- or -CH₂-, when Z is -O- and x indicates an integer of 4 to 16, y indicates an integer of 26 to 35, when Z is -O- and x indicates an integer of 17 to 25, y indicates an integer of 0 to 35, when Z is -CH₂- and x indicates an integer of 4 to 15, y indicates an integer of 26 to 35, and Z is -CH₂- and x indicates an integer of 16 to 25, y indicates an integer of 0 to 35.

2. A glycolipid derivative as claimed in claim 1 wherein, in the formula (I), Z indicates -O-, x indicates an integer of 4 to 16 and y indicates an integer of 26 to 35.

3. A glycolipid derivative as claimed in claim 1 wherein, in the formula (I), Z indicates -O-, x indicates an integer of 17 to 25 and y indicates an integer of 0 to 35.

4. A glycolipid derivative as claimed in claim 1 wherein, in the formula (I), Z indicates -O-, x indicates an integer of 17 to 25 and y indicates an integer of 20 to 28.

5. A glycolipid derivative as claimed in claim 1 wherein, in the formula (I), Z indicates -CH₂-, x indicates an integer of 4 to 15 and y indicates an integer of 26 to 35.

6. A glycolipid derivative as claimed in claim 1 wherein, in the formula (I), Z indicates -CH₂-, x indicates an integer of 16 to 25 and y indicates an integer of 0 to 35.

7. A glycolipid derivative as claimed in claim 1 wherein, in the formula (I), Z indicates -CH₂-, x indicates an integer of 16 to 25 and y indicates an integer of 20 to 28.

8. A glycolipid derivative as claimed in claim 1 wherein, in the formula (I), R¹ indicates α-D-galactopyranosyl.

9. A glycolipid derivative as claimed in claim 1 wherein, in the formula (I), R¹ indicates α-D-galactopyranosyl, Z indicates -O-, x indicates an integer of 4 to 16 and y indicates an integer of 26 to 35.

10. A glycolipid derivative as claimed in claim 1 wherein, in the formula (I), R¹ indicates α-D-galactopyranosyl, Z indicates -O-, x indicates an integer of 17 to 25 and y indicates an integer of 0 to 35.

11. A glycolipid derivative as claimed in claim 1 wherein, in the formula (I), R¹ indicates α-D-galactopyranosyl, Z indicates -O-, x indicates an integer of 17 to 25 and y indicates an integer of 20 to 28.

12. A glycolipid derivative as claimed in claim 1 wherein, in the formula (I), R¹ indicates α-D-galactopyranosyl, Z indicates -CH₂-, x indicates an integer of 4 to 15 and y indicates an integer of 26 to 35.

13. A glycolipid derivative as claimed in claim 1 wherein, in the formula (I), R¹ indicates α-D-galactopyranosyl, Z indicates -CH₂-, x indicates an integer of 16 to 25 and y indicates an integer of 0 to 35.

14. A glycolipid derivative as claimed in claim 1 wherein, in the formula (I), R¹ indicates α-D-galactopyranosyl, Z indicates -CH₂-, x indicates an integer of 16 to 25 and y indicates an integer of 20 to 28.

15. A glycolipid derivative as claimed in claim 1 wherein, in the formula (I), R¹ indicates α-D-galactopyranosyl, A indicates -CH₂- or -CH(OH)-CH₂-, Z indicates -O-, x indicates an integer of 4 to 16 and y indicates an integer of 26 to 35.

16. A glycolipid derivative as claimed in claim 1 wherein, in the formula (I), R¹ indicates α-D-galactopyranosyl, A indicates -CH₂- or -CH(OH)-CH₂-, Z indicates -O-, x indicates an integer of 17 to 25 and y indicates an integer of 0 to 35.

17. A glycolipid derivative as claimed in claim 1 wherein, in the formula (I), R¹ indicates α-D-galactopyranosyl, A indicates -CH₂- or -CH(OH)-CH₂-, Z indicates -O-, x indicates an integer of 17 to 25 and y indicates an integer of 20 to 28.

18. A glycolipid derivative as claimed in claim 1 wherein, in the formula (I), R¹ indicates α-D-galactopyranosyl, A indicates -CH₂- or -CH(OH)-CH₂-, Z indicates -CH₂-, x indicates an integer of 4 to 15 and y indicates an integer of 26 to 35.

19. A glycolipid derivative as claimed in claim 1 wherein, in the formula (I), R¹ indicates α-D-galactopyranosyl, A indicates -CH₂- or -CH(OH)-CH₂-, Z indicates -CH₂-, x indicates an integer of 16 to 25 and y indicates an integer of 0 to 35.

20. A glycolipid derivative as claimed in claim 1 wherein, in the formula (I), R¹ indicates α-D-galactopyranosyl, A indicates -CH₂- or -CH(OH)-CH₂-, Z indicates -CH₂-, x indicates an integer of 16 to 25 and y indicates an integer of 20 to 28.

21. A glycolipid derivative as claimed in claim 1 wherein, in the formula (I), R¹ indicates α-D-galactopyranosyl, R² indicates a hydrogen atom, A indicates -CH₂- or -CH(OH)-CH₂-, Z indicates -O-, x indicates an integer of 4 to 16 and y indicates an integer of 26 to 35.

22. A glycolipid derivative as claimed in claim 1 wherein, in the formula (I), R¹ indicates α-D-galactopyranosyl, R² indicates a hydrogen atom, A indicates -CH₂- or -CH(OH)-CH₂-, Z indicates -O-, x indicates an integer of 17 to 25 and y indicates an integer of 0 to 35.

23. A glycolipid derivative as claimed in claim 1 wherein, in the formula (I), R¹ indicates α-D-galactopyranosyl, R² indicates a hydrogen atom, A indicates -CH₂- or -CH(OH)-CH₂-, Z indicates -O-, x indicates an integer of 17 to 25 and y indicates an integer of 20 to 28.

24. A glycolipid derivative as claimed in claim 1 wherein, in the formula (I), R¹ indicates α-D-galactopyranosyl, R² indicates a hydrogen atom, A indicates -CH₂- or -CH(OH)-CH₂-, Z indicates -CH₂-, x indicates an integer of 4 to 15 and y indicates an integer of 26 to 35.

25. A glycolipid derivative as claimed in claim 1 wherein, in the formula (I), R¹ indicates α-D-galactopyranosyl, R² indicates a hydrogen atom, A indicates -CH₂- or -CH(OH)-CH₂-, Z indicates -CH₂-, x indicates an integer of 16 to 25 and y indicates an integer of 0 to 35.

26. A glycolipid derivative as claimed in claim 1 wherein, in the formula (I), R¹ indicates α-D-galactopyranosyl, R² indicates a hydrogen atom, A indicates -CH₂- or -CH(OH)-CH₂-, Z indicates -CH₂-, x indicates an integer of 16 to 25 and y indicates an integer of 20 to 28.

27. A glycolipid derivative as claimed in claim 1 wherein, in the formula (I), R¹ indicates α-D-galactopyranosyl, R² indicates a hydrogen atom, A indicates -CH(OH)-CH₂-, Z indicates -O-, x indicates an integer of 4 to 16 and y indicates an integer of 26 to 35.

28. A glycolipid derivative as claimed in claim 1 wherein, in the formula (I), R¹ indicates α-D-galactopyranosyl, R² indicates a hydrogen atom, A indicates -CH (OH) -CH₂-, Z indicates -O-, x indicates an integer of 17 to 25 and y indicates an integer of 0 to 35.

29. A glycolipid derivative as claimed in claim 1 wherein, in the formula (I), R¹ indicates α-D-galactopyranosyl, R² indicates a hydrogen atom, A indicates -CH(OH)-CH₂-, Z indicates -O-, x indicates an integer of 17 to 25 and y indicates an integer of 20 to 28.

30. A glycolipid derivative as claimed in claim 1 wherein, in the formula (I), R¹ indicates α-D-galactopyranosyl, R² indicates a hydrogen atom, A indicates -CH(OH)-CH₂-, Z indicates -CH₂-, x indicates an integer of 4 to 15 and y indicates an integer of 26 to 35.

31. A glycolipid derivative as claimed in claim 1 wherein, in the formula (I), R¹ indicates α-D-galactopyranosyl, R² indicates a hydrogen atom, A indicates -CH(OH)-CH₂-, Z indicates -CH₂-, x indicates an integer of 16 to 25 and y indicates 0 to 35.

32. A glycolipid derivative as claimed in claim 1 wherein, in the formula (I), R¹ indicates α-D-galactopyranosyl, R² indicates a hydrogen atom, A indicates -CH(OH)-CH₂-, Z indicates -CH₂-, x indicates an integer of 16 to 25 and y indicates an integer of 20 to 28.

33. A drug for treatment of an autoimmune disease comprising, as an active component, a glycolipid derivative according to any one of claims 1 to 32.

34. A drug for treatment of an allergic disease comprising, as an active component, a glycolipid derivative according to any one of claims 1 to 32.

35. A drug for treatment of a disease, in which NKT cells or stimulus to NKT cells is participated in the deterioration of the disease conditions comprising, as an active component, a glycolipid derivative according to any one of claims 1 to 32.
